# EUROPEAN PATENT SPECIFICATION

(11) **EP 3 368 052 B1**
(45) Date of publication and mention of the grant of the patent: **08.12.2021**
(21) Application number: 16860828.9
(22) Date of filing: 27.10.2016
(51) Int. Cl.: A61K 35/28, A61K 35/14, C12N 15/85, C12N 5/0789

(54) **USE OF MAPK INHIBITORS TO REDUCE LOSS OF HEMATOPOIETIC STEM CELLS DURING EX VIVO CULTURE AND GENETIC MANIPULATION**
VERWENDUNG VON MAPK-INHIBITOREN ZUR VERRINGERUNG DES VERLUSTS HÄMATOPOETISCHER STAMMZELLEN WÄHREND DER EX-VIVO-KULTIVIERUNG UND GENETISCHEN MANIPULATION
UTILISATION D'INHIBITEURS DE MAPK POUR RÉDUIRE LA PERTE DE CELLULES SOUCHES HÉMATOPOÏÉTIQUES PENDANT LA CULTURE EX VIVO ET LA MANIPULATION GÉNÉTIQUE

(30) Priority: 27.10.2015 US 201562246964 P
(43) Date of publication of application: 05.09.2018
(73) Proprietor: Children's Hospital Medical Center, Cincinnati, Ohio 45229-3029 (US)
(72) Inventor: MALIK, Punam, Cincinnati, OH 45220 (US); FILIPPI, Marie-dominique, Cincinnati, OH 45242 (US)
(74) Representative: Cabinet Becker et Associés
(86) International application number: PCT/US2016/059204
(87) International publication number: WO 2017/075274

(56) References cited:
- WO-A2-2012/141971
- US-A1- 2013 171 110
- US-A1- 2015 166 969
- JING ZOU ET AL: "Inhibition of p38 MAPK activity promotes ex vivo expansion of human cord blood hematopoietic stem cells", ANNALS OF HEMATOLOGY, vol. 91, no. 6, 19 January 2012 (2012-01-19), pages 813-823, XP055562587, DE ISSN: 0939-5555, DOI: 10.1007/s00277-011-1397-7
- KEISUKE ITO ET AL: "Reactive oxygen species act through p38 MAPK to limit the lifespan of hematopoietic stem cells", NATURE MEDICINE, vol. 12, no. 4, 26 March 2006 (2006-03-26) , pages 446-451, XP055203670, ISSN: 1078-8956, DOI: 10.1038/nm1388
- YI LUO ET AL: "Rapamycin Enhances Long-Term Hematopoietic Reconstitution of Ex Vivo Expanded Mouse Hematopoietic Stem Cells by Inhibiting Senescence :", TRANSPLANTATION, vol. 97, no. 1, 1 January 2014 (2014-01-01), pages 20-29, XP055562585, GB ISSN: 0041-1337, DOI: 10.1097/TP.0b013e3182a7fcf8

## Description

### BACKGROUND

Hematopoietic stem cells (HSC) are a unique and rare population of cells that have the ability to reconstitute the whole hematopoietic system and to undergo self-renewal for the maintenance of their population. Gene therapy (GT), which involves transferring a gene into HSCs, offers an attractive treatment strategy for curing hematopoietic disorders. However, GT requires *ex vivo* manipulation and culture of HSCs largely results in a large amount of HSC loss as they differentiate into hematopoietic progenitor cells (HPCs).

Currently, the numbers of human HSCs that repopulate after autologous transplants are a major limitation to effective gene transfer. Myelo-ablative conditioning is therefore often required to destroy resident HSCs, giving an engraftment advantage to the limited numbers of genetically-manipulated HSCs following *ex vivo* manipulation.

There is a lack of a good experimental model of human HSCs other than non-human primate models, which have limitations with respect to availability, the numbers of animals that can be transplanted, and high costs.

Immune deficient mice, specifically the NOD/SCID/IL-2Rgnull (NSG mice), can robustly engraft human CD34⁺ HSC/P, giving rise to B, Myeloid and T cell progeny, and have been used to study human hematopoiesis *in vivo.* However, most studies in NSG mice use umbilical cord blood CD34⁺ HSC/P that engraft readily but do not mimic bone marrow (BM) or mobilized peripheral blood (MPB) derived HSC/P kinetics or engraftment. The latter are primarily used for gene therapy.

Thus, development of a robust model of human HSC (not HPC) engraftment that can be used to test HSC maintenance during *ex vivo* manipulation will greatly advance the field of gene therapy.

### SUMMARY

The present disclosure is based, at least in part, on the unexpected discovery that inhibiting activation of p38 mitogen-activated protein kinase (MAPK) in stem cells, for example, using a p38 MAPK inhibitor such as doramapimod, successfully reduced the loss of stem cells during *in vitro* or *ex vivo* culture wherein the stem cells have undergone genetic manipulations that induce a DNA double strand break, such as vector integration, thereby increasing engraftment of the cultured stem cells *in vivo.*

The invention is as defined in the appended claims.

Accordingly, one aspect of the present disclosure features a method for preparing stem cells such as hematopoietic stem cells (HSCs) having enhanced engraftment activity. The method comprises culturing stem cells *(e.g.,* HSCs) that have undergone a genetic manipulation in a culture medium comprising an effective amount of a p38 MAPK inhibitor. The genetic manipulation induces a DNA double strand break in the stem cells. For example, the stem cells that are amenable to the methods described herein can be non-cycling or non-dividing stems cells (resting cells).

In another aspect, the present disclosure provides a method for preparing human stem cells such as hematopoietic stem cells (HSCs) having enhanced engraftment capacity, the method comprising: culturing human adult stem cells *(e.g.,* human adult HSCs) in a medium that comprises an effective amount of a p38 MAPK inhibitor. In some examples, the human adult stem cells *(e.g.,* human adult HSCs) are non-cycling or non-dividing human adult stem cells.

Any of the methods described herein comprise, prior to the culturing step, genetically manipulating the stem cells. The genetic manipulation may comprise transducing the stem cells with an integrating vector or performing genome editing in the stem cells. The genome editing may involve use of, *e.g.,* but not limited to CRISPR-Cas9 systems, zinc finger nucleases (ZFN), and/or transcription activator-like effector-based nucleases (TALEN). The genetic manipulation is performed prior to a cell division cycle, more particularly in quiescent G0 phase. Examples of an integrating vector include, but are not limited to viral vectors such as lentiviral vector and retroviral vectors.

The HSCs that have been cultured in the presence of a p38 MAPK inhibitor are useful for administering or transplanting to a subject in need thereof. The HSCs may be cultured for 1 to 7 days prior to their administration or transplantation into the subject.

The HSCs are useful in a method of treating a hematopoietic disorder in a subject in need of the treatment. The method comprises: (a) providing HSCs that have been genetically manipulated and cultured in a medium comprising an effective amount of a p38 for at least 24 hours or longer, (b) transplanting a first population of the HSCs to the subject after (a), and optionally (c) transplanting a second population of the HSCs to the subject after (b).

In any methods described herein, the stem cells *(e.g.,* HSCs) can be derived from bone marrow, peripheral blood cells, and/or umbilical cord blood of a suitable source *(e.g.,* human). The stem cells *(e.g.,* HSCs) can be allogeneic stem cells *(e.g.,* HSCs) or autologous stem cells *(e.g.,* HSCs).

In any aspects described herein, the p38 MAPK inhibitor can be a protein, a nucleic acid, a small molecule, or a combination thereof. In some examples, the p38 MAPK inhibitor can be a p38 MAPK blocking agent *(e.g.,* a small molecule that binds p38-α and blocks p38 MAPK signaling). Examples of the p39 MAPK inhibitor include, but are not limited to doramapimod *(e.g.,* BIRB-796), ralimetinib *(e.g.,* LY2228820 dimesylate), aminopyridine-based, ATP-competitive inhibitors of p38 MAPK (*e.g.*, Vx702), pyridinyl imidazole inhibitors (*e.g*., SB203580), and any combinations thereof.

In any of the methods described herein, the amount of the p38 MAPK inhibitor can be effective to increase the proportion of stem cells *(e.g.,* HSCs) in the G0 quiescent phase and to decrease the proportion of the stem cells *(e.g.,* HSCs) in the S-G2-M phase before the first cell division cycle *(e.g.,* 24 hours); to delay the transition of stem cells from G0 quiescent phase to S phase; to reduce loss of long term repopulating potential (LTRP) in the stem cells; to reduce the myeloid skewing phenotype in the stem cells; and/or to promote engraftment of the HSCs transplanted to the subject.

In any of the methods described herein, the subject can be a human subject. In some embodiments, the subject is a human patient having a hematopoietic disorder.

In some embodiments, the HSCs can be human adult HSCs, which may be obtained from the bone marrow or peripheral blood cells of a human subject. The HSCs are cultured *ex vivo* prior to step (ii). The HSCs undergo a genetic manipulation that induces a DNA double strand break in the *ex vivo* culture. The genetic manipulation can be transduction of an integrating vector, for example, a viral vector such as a retroviral vector or a lentiviral vector. When the HSCs have been transduced by a lentiviral vector, the HSCs can be transplanted to the animal after 18-96 hours of *ex vivo* culture. When the HSCs have been transduced by a retroviral vector, the HSCs can be transplanted to the animal after 72-96 hours of *ex vivo* culture. The genetic manipulation may comprise performing genome editing in the stem cells, e.g., using any methods known in the art including, e.g., CRISPR-Cas9 systems, zinc finger nucleases (ZFN), and/or transcription activator-like effector-based nucleases (TALEN).

### BRIEF DESCRIPTION OF THE DRAWINGS

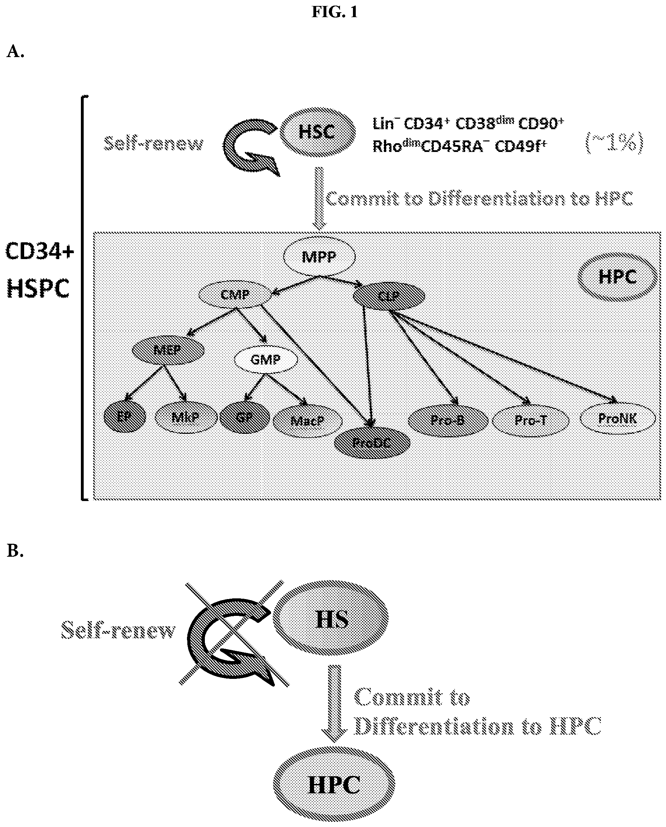

Fig. 1 shows the possible fate of hematopoietic stem cells. Panel A is a schematic illustrating that human CD34+ cells comprise the hematopoietic stem and progenitor compartment, which includes hematopoietic stem cells (HSCs) and hematopoietic progenitor cells (HPCs). HSC fate decision is to either self renew or commit to differentiation into HPC. Panel B shows the hypothesis that genetic manipulation alters HSC fate to HPCs.

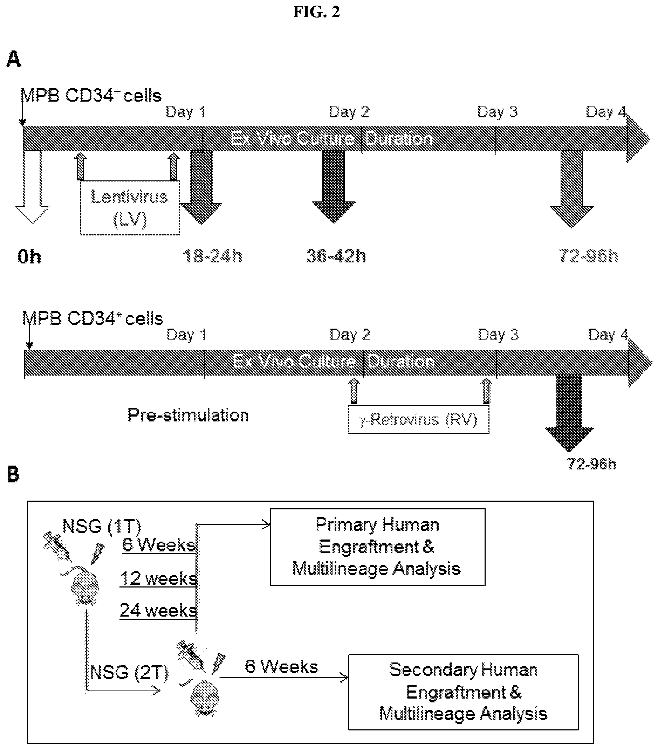

Fig. 2. A preclinical model for studying adult human HSC ex vivo manipulation and gene transfer and its effect on LTRP. (Panel A) Freshly isolated or thawed mobilized peripheral blood (MPB) derived CD34+ cells were utilized for the study. Two different protocols were utilized for the study where lentivirus (LV) or γ-Retrovirus (RV) mediated gene transfer was performed at indicated time points. 0h, 18-24h, 36-42h, and 72-96h indicates the total amount of time where cells were exposed in the ex vivo culture after CD34 isolation before injection into NSG mice. (Panel B) After indicated time in culture and transduction, 1 million CD34+ starting equivalent cells were transplanted per NSG mouse after TBI intravenously (primary transplant = 1T). Primary human engraftment and multilineage reconstitution was analyzed in mice at indicated time periods after bone marrow harvest at 6 and 12 weeks (wks) from left and right femurs, and after sacrifice at 24 wks, from all bones. A portion of cells were analyzed for FACS and the rest were depleted of mouse CD45+ cells and transplanted one to one, into secondary mice (secondary transplant = 2T).

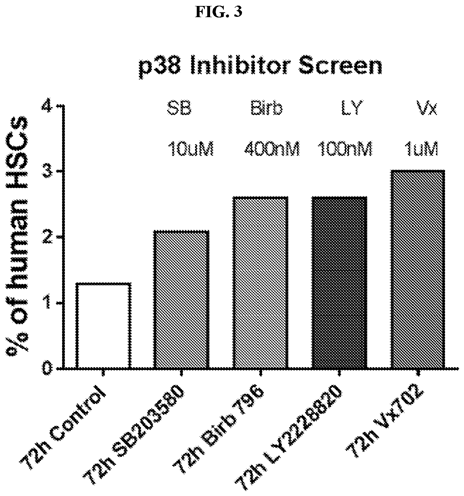

Fig. 3 shows that p38 inhibitors prevent HSC loss and may even retain HSC through cell division during *ex vivo* culture. Four different p38 inhibitors all increase the percent hHSCs *ex vivo* in culture studies up to 72 hours.

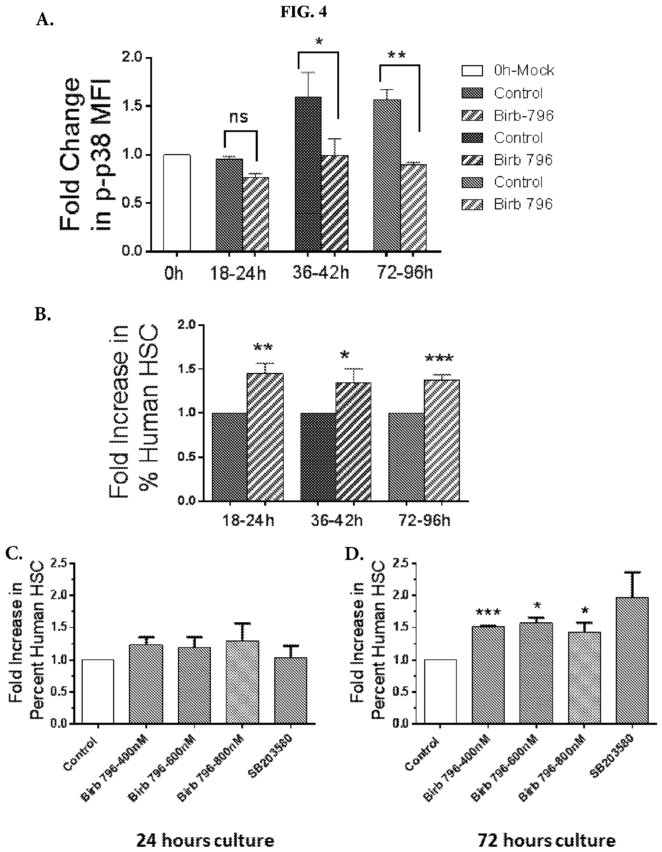

Fig. 4 shows BIRB-796 significantly decreases p-p38 in hHSCs in *ex vivo* cultures (panel A). Panel B shows an analysis of percent hHSC in cultured CD34⁺ cells at the indicated time interval with or without BIRB-796. Panels C and D show that p38 inhibitor BIRB-796 appeared to increase human HSCs *in vitro,* at 400, 600 and 800nm concentrations.

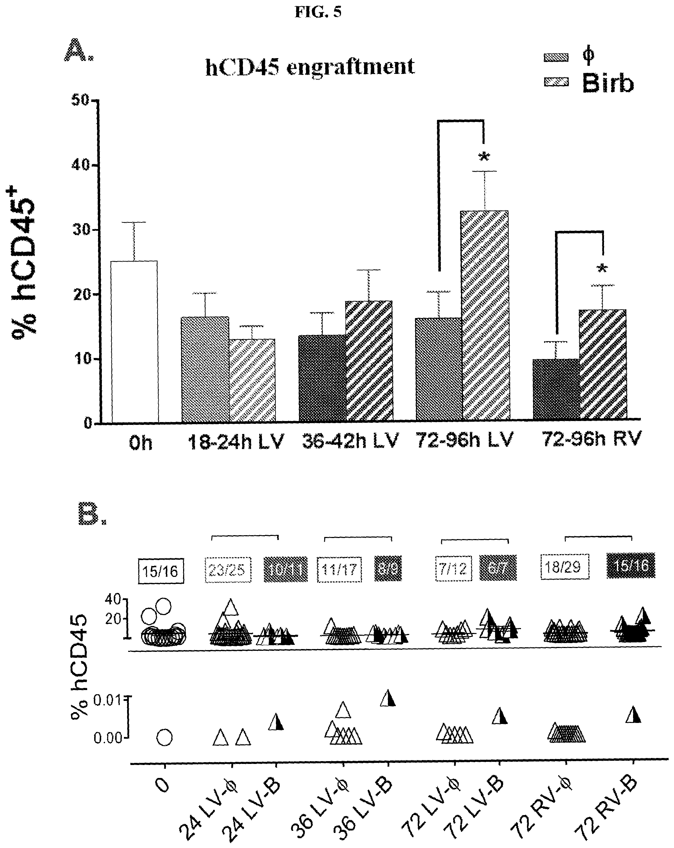

Fig. 5 shows p38 inhibition significantly increases human engraftment in primary transplanted mice and also increases the number of 2T mice with human cell engraftment. Panel A: hCD45⁺ cell engraftment in bone marrow of 1T mice at 24 weeks following transplant of cultured hCD34⁺ cells with/without p38 inhibitor Birb796. Panel B: Number of 2T mice engrafted (>0.01% hCD45⁺ cells in BM) are shown above the middle bar and those non-engrafted below. Each symbol represents one mouse and the total numbers are indicated. φ=control, B=BIRB-796, 0, 24, 36 and 72 indicate hours of *ex vivo* culture while transduced with Lentivirus (LV) or Retrovirus (RV) vector encoding GFP.

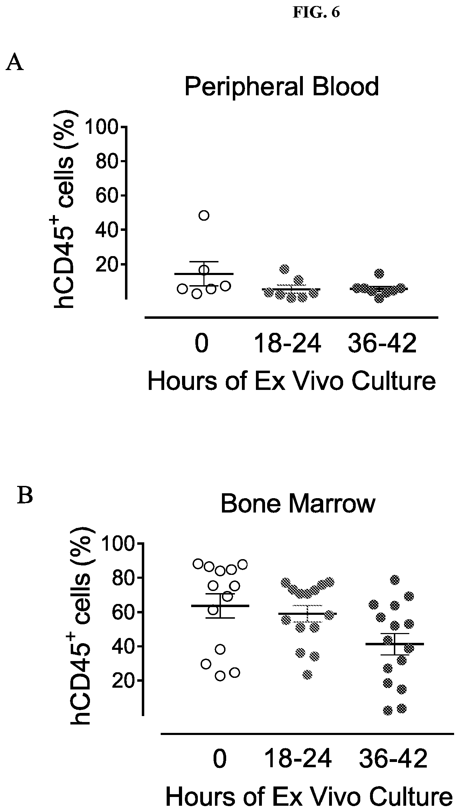

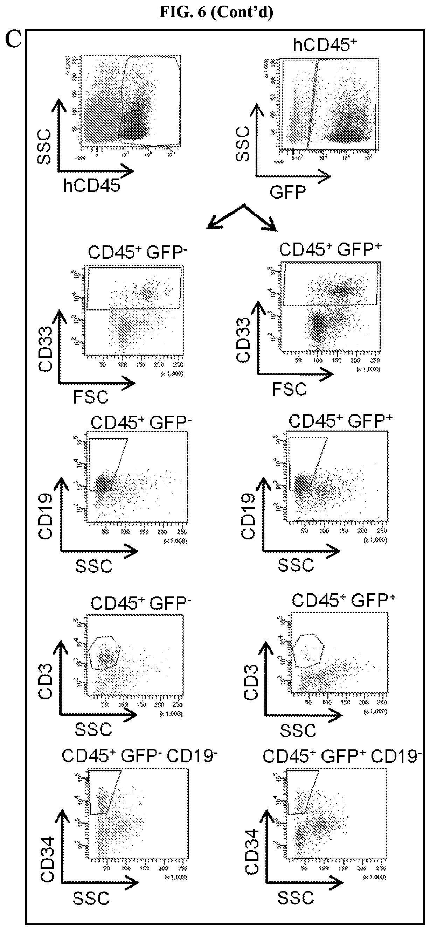

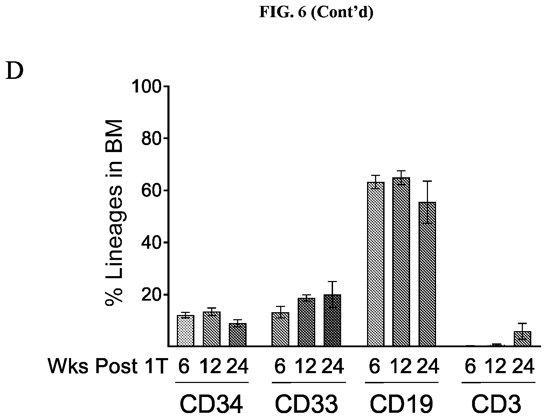

Fig. 6 shows a human xenograft model of adult hematopoietic stem cells. Freshly isolated human MPB derived CD34⁺ cells (1 million CD34⁺ cells/mouse) were transduced with a lentivirus vector within 24 hours and injected in lethally irradiated NSG mice intravenously. Primary human engraftment was analyzed in mice at 6 weeks, both in bone marrow (BM) (Panel A) and in peripheral blood (PB) (Panel B). Each symbol represents an individual mouse and plotted as mean ± S.E.M. (Data are representative of BM: 0h n=13, 18-24h n=14, 36-42h n=15; PB: 0h n=6, 18-24h n=7, 36-42h n=8 mice). Panel C: At 6, 12 and 24 weeks post primary transplant (1T), BM was analyzed for the different human cell populations by flow cytometry . Representative FACS plots shows the total human CD45⁺ (CD45⁺) cells followed by gating of GFP⁺ (transduced) versus GFP- (untransduced) cells. From the GFP⁻ and GFP⁺ human CD45⁺ populations, human CD33⁺ myeloid cells, human CD19⁺ B-Lymphoid and CD3⁺ T-Lymphoid cells and human CD34⁺ HSPCs, that were negative for CD19 were analyzed. FACS plot shown from 24 week post 1T is shown in Panel C. The percent multi-lineage engraftment analyzed at 6, 12, and 24 weeks after primary transplantation (1T) of uncultured MPB derived CD34⁺ cells is shown in Panel D, plotted as mean± S.E.M. Data are representative of 6wks n=20, 12 wks n=12, 24wks n=10 mice).

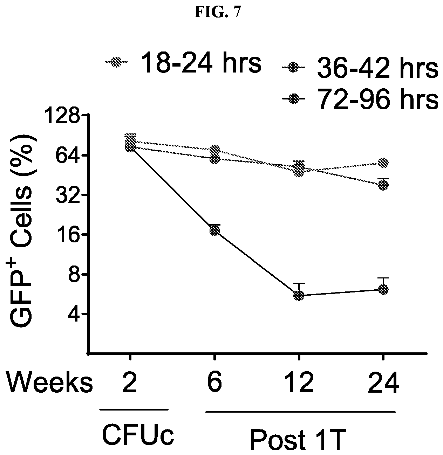

Fig. 7 shows GFP marking *in vitro* and *in vivo.* Human MPB CD34⁺ cells were cultured and transduced with the LV or RV for the indicated hours as described in Fig. 2 (Panel A), and colony forming unit cells (CFUc) plated on a small proportion of CD34+ cells, while the rest (1 million starting equivalent) were transplanted IV into NSG mice. BM was aspirated at 6 and 12 weeks from right and left femurs, and mice sacrificed at 24 weeks; BM was analyzed for GFP⁺ hCD45⁺ cells at 6, 12 and 24 weeks (for 18-24h n=20, for 36-42h n=9, and for 72-96h n= 7 for the total of 36 mice; data presented as mean ± SEM).

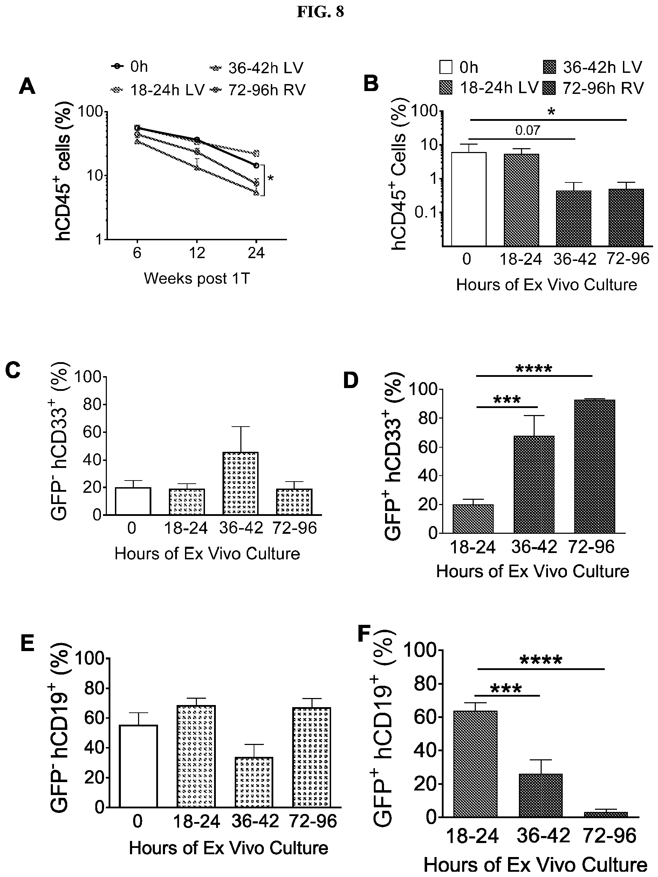

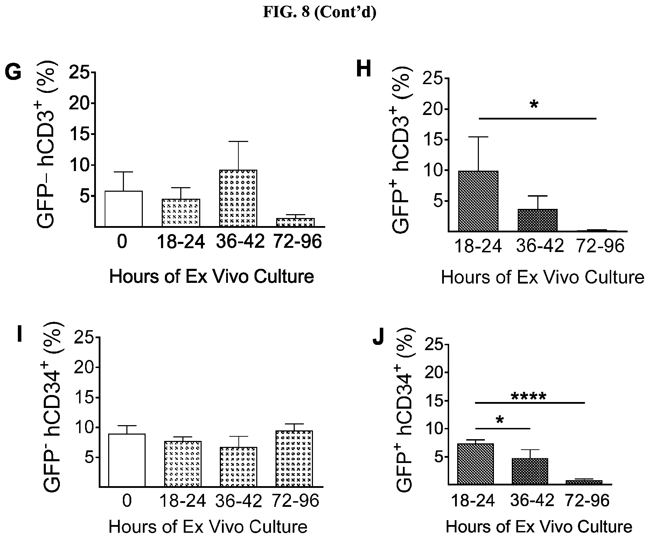

Fig. 8 shows *ex vivo* manipulation and gene transfer in human MPB CD34⁺ HSPC for longer than 24h results in significant loss of LTRP and a myeloid-skewed gene-modified progeny. Panel A: Freshly isolated or thawed human MPB derived CD34⁺ cells were cultured and transduced with lentivirus vector (LV) or γ-retrovirus vector (RV) for the indicated hours. An equivalent input of one million CD34⁺ cells were injected per irradiated NSG mouse. The percentage of human CD45⁺ cells in BM of primary NSG mice at the indicate time points is shown. The x-axis denotes the number of weeks after primary transplant (1T). Data are representative of: for 0h mock n=10, for 18-24h n= 20, for 36-42h n= 9, for 72-96h n= 19 mice. Panel B: Total human cells in BM of primary NSG mice were transplanted one to one into irradiated secondary mice. Human engraftment in bone marrow (Y-axis) 6 weeks post secondary transplant (2T) is shown (for 0h mock n=7, for 18-24h n= 5, for 36-42h n= 14, for 72-96h n= 14 for the total of 40 mice. Multi-lineage reconstitution was analyzed 24 weeks post 1T. Both untransduced (GFP⁻) and transduced (GFP⁺) human CD33⁺ myeloid population (Panels C and D), human CD19⁺ B-Lymphoid (Panel E, Panel F), human CD3⁺ T-Lymphoid (Panels G and H), and human CD34⁺ HSPCs population (Panels I and J) are shown (n=17-22 per condition). Data expressed as mean ± SEM. Statistics: Mann Whitney U test, ^{∗∗∗∗} P<0.0001, ^{∗∗∗} P<0.001, ^{∗∗}P<0.01,^{∗}P<0.05.

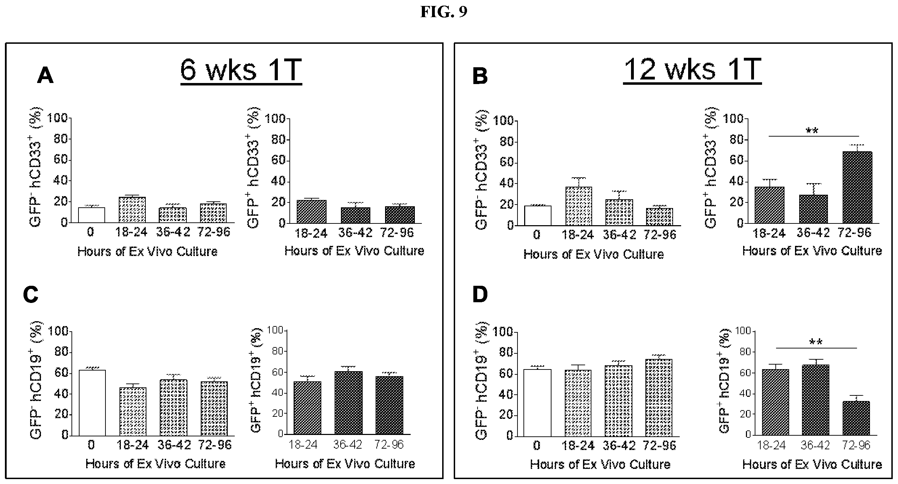

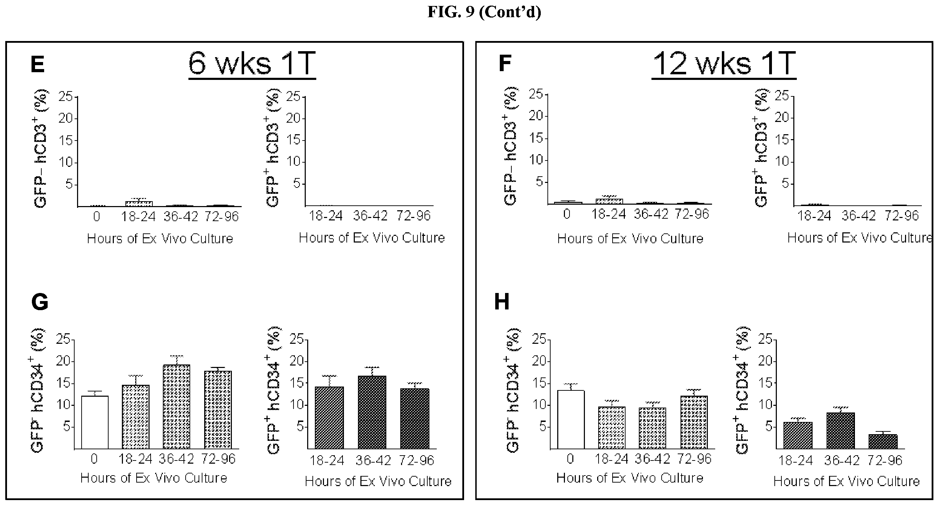

Fig. 9 shows multi-lineage engraftment of *ex vivo* manipulated and cultured MPB derived hCD34⁺ cells in NSG mice. Human MPB CD34⁺ cells were cultured for the indicated hours (X axes), and transduced with lentivirus vector (LV) or γ-retrovirus vector (RV) encoding enhanced green fluorescent protein (GFP) as a marker of transduced cells; an equivalent input of one million CD34⁺ cells were injected per irradiated (280cGy) NSG mouse thereafter. Bone marrow was analyzed for multi-lineage reconstitution at 6 wks (Panels A, C, E, and G) and 12 wks (Panels B, D, F, and H) after primary transplant. Both untransduced (GFP⁻) and transduced (GFP⁺) human CD33⁺ myeloid population (Panels A and B), human CD19⁺ B-Lymphoid (Panels C and D), human CD3⁺ T-Lymphoid (Panels E and F), and human CD34⁺ HSPCs population (Panels G and H) are shown (Data are representative of 0h n= 12, 18-24h n= 7, 36-42h n= 11, 72-96h n= 20 for the total of 50 NSG mice). Data expressed as mean ± SEM. Statistics: Mann Whitney test, ^{∗∗} P<0.01.

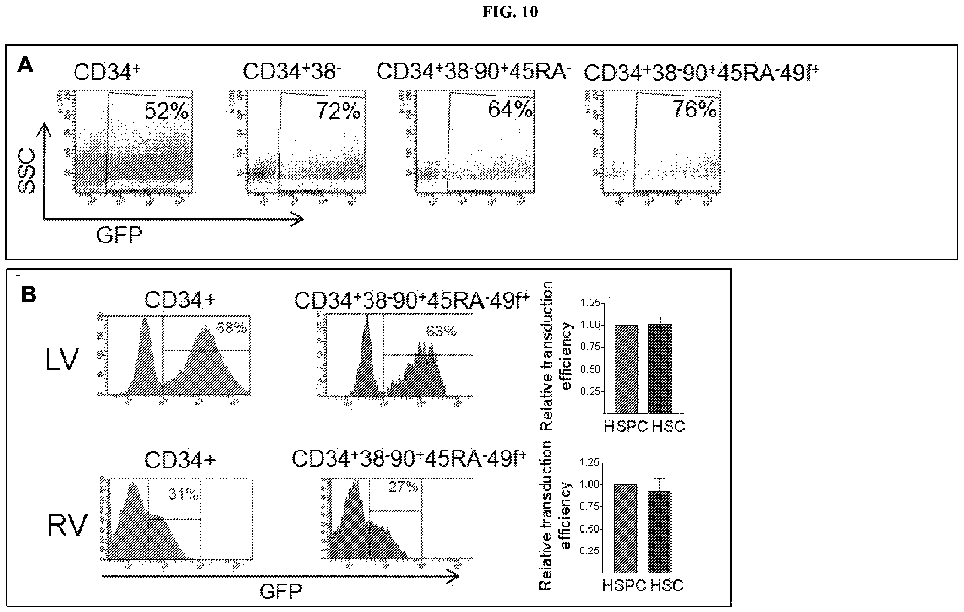

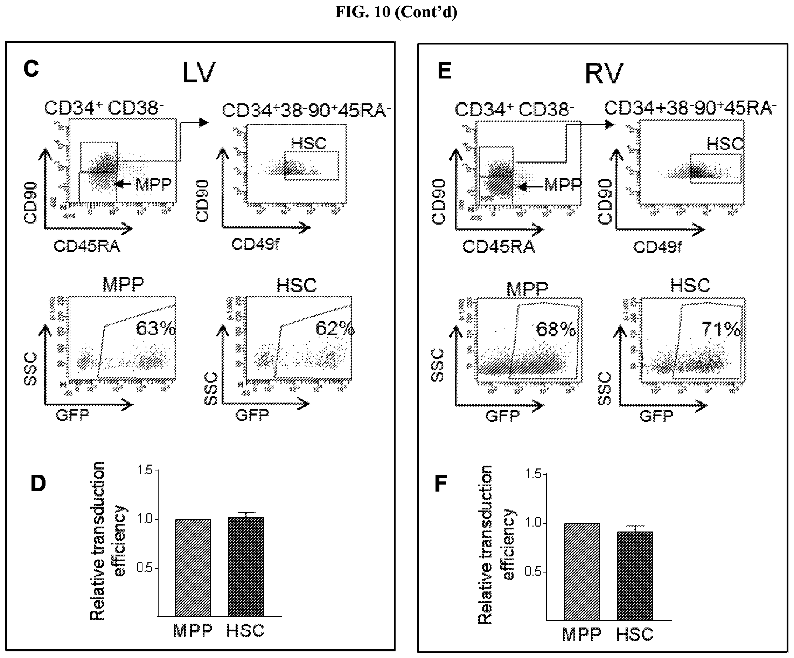

Fig. 10 shows that both lentiviral (LV) and retroviral (RV) vectors transduce human hematopoietic progenitor cells (HPCs) and hematopoietic stem cells (HSCs) comparably. Representative fluorescence-activated cell sorting (FACS) plot showing green fluorescent protein (GFP) marker expression in various hematopoietic stem/progenitor (HSPC) subsets after LV transduction for 42 hours (Panel A), in human CD34⁺ hematopoietic stem/progenitor cells (HSPCs) versus CD34⁺ 38⁻ 90⁺ 45RA⁻ 49f⁺ HSCs post LV or RV transduction for 72 hours (left) and the quantification (right) (Panel B) (n=3 independent experiments) and in MPP versus HSC after LV (Panel C) or RV (Panel D) transduction for 72 hours along with the quantification of the respective groups (Panels E and F). Bar graph with mean ± SEM of LV transduction (Panel E) or RV transduction (Panel F) is shown (n=3 independent experiments).

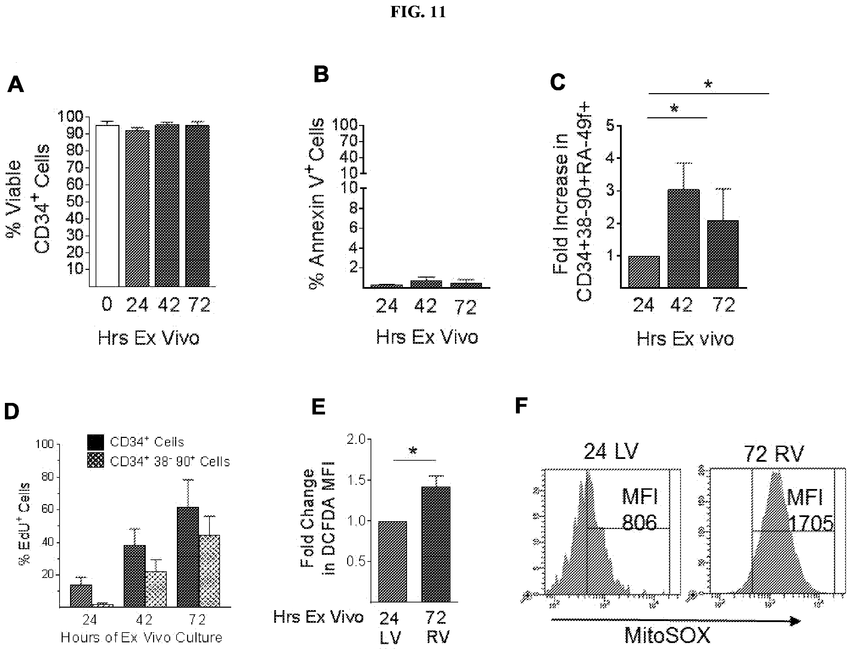

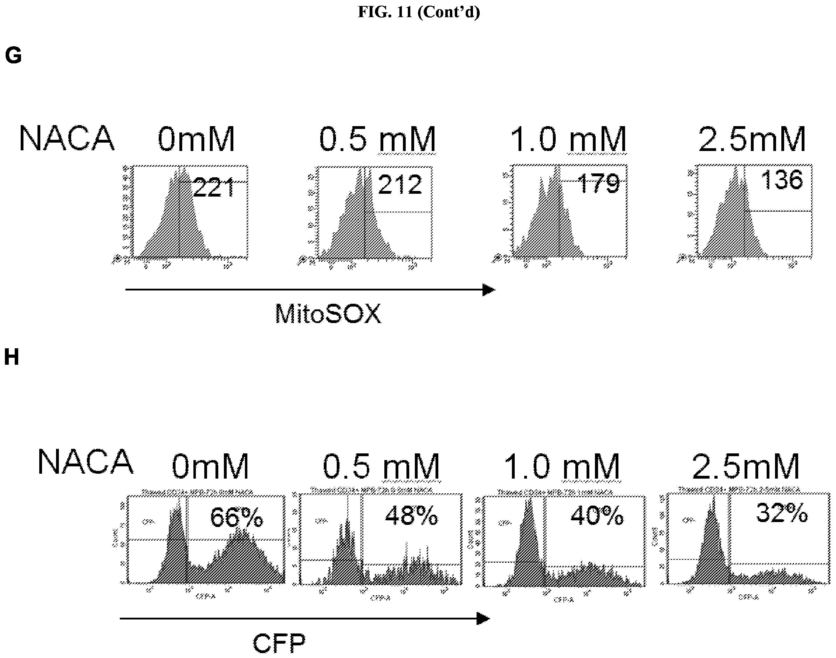

Fig. 11 shows that *ex vivo* manipulation is not associated with reduced viability or apoptosis of HSC, but with increased phenotypic HSC with higher ROS; reducing ROS decreases gene transfer. Human MPB derived CD34⁺ cells were cultured and transduced with lentivirus (LV) or retrovirus (RV) for the indicated time points. Panel A: Total cell viability after harvest was determined by trypan blue exclusion method. Panel B: Annexin V⁺ (apoptotic) CD34⁺ 38⁻ 90⁺ cells after *ex vivo* culture was detected by flow cytometry. Panel C: Fold increase in phenotypic human HSCs (CD34⁺ 38⁻ 90⁺ 45RA⁻ 49f⁺ cells) was calculated after flow cytometric analysis (n=3). Statistics: Student's t test, ^{∗}p<0.05. Panel D: Proliferation status of human CD34⁺ HSPC versus CD34⁺ 38⁻ 90⁺ HSC enriched population during *ex vivo* culture determined by the proportion of EdU⁺ HSPCs and HSCs in the EdU incorporation assay (n=3). Panel E: Intracellular ROS levels were determined using CM-H2DCFDA fluorescence; fold change in DCFDA MFI in LV and RV transduced human CD34⁺ 38⁻ 90⁺ cells is shown as mean of ± SEM (n=3). Statistics: Student's t test, ^{∗}p<0.05. Panel F: Representative histogram plots showing MitoSOX for the measurement of mitochondrial-specific ROS in human CD34+ 38- 90+ cells cultured for 24 hours versus 72 hours. The numbers represents MFI. Panel G: Histogram plots showing MitoSOX levels with increasing doses of the anti-oxidant N-acetylcysteine amide (NACA) and Panel H: corresponding percentage of CFP+ CD34+ 38- 90+ cells measured by flow cytometry.

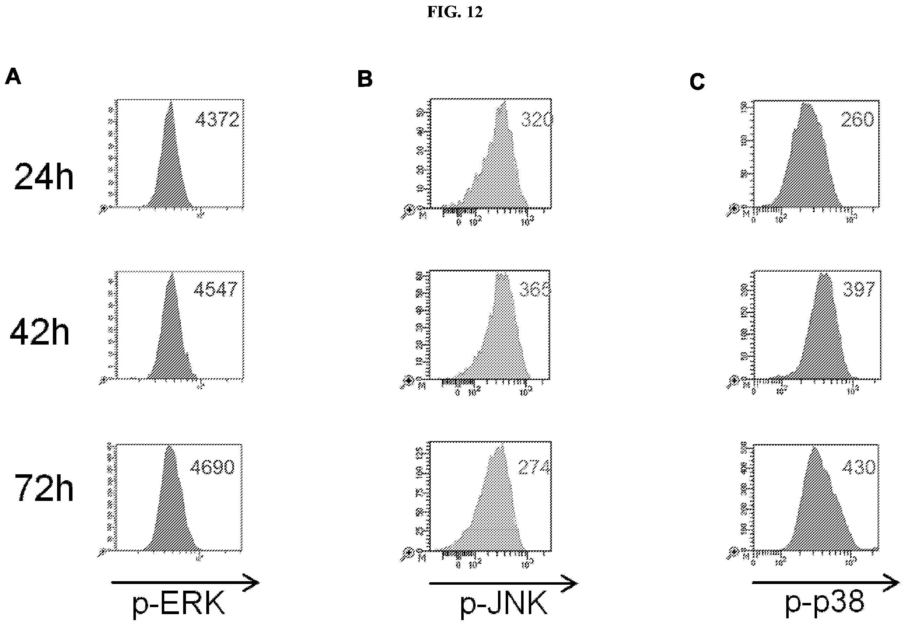

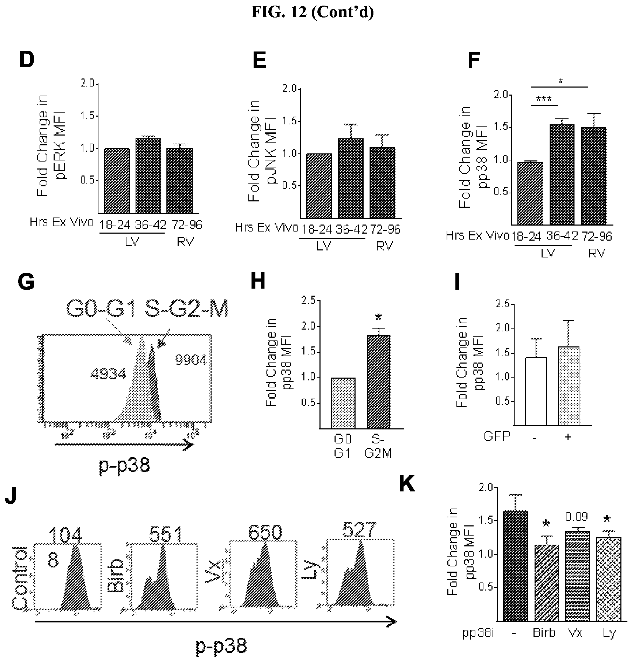

Fig. 12 shows increase in time in culture activates p38 MAPK in HSC. Human MPB derived CD34⁺ cells were cultured and transduced as described in Fig. 7. Representative histograms plots showing phospho-ERK (p-ERK) (Panel A), phospho JNK (p-JNK) (Panel B), and phospho-p38MAPK (p-p38) (Panel C) levels in human CD34⁺ 38⁻ 90⁺ HSC-enriched population by flow cytometry. Quantitative fold-change in mean fluorescent intensity (MFI) of p-ERK (Panel D), p-JNK (Panel E) and p-p38 (Panel F) at indicated times are shown. Data expressed as mean ± SEM from 3 independent experiments using three different MPB donors. Statistics: Student's t test, ^{∗}p<0.05, ^{∗∗}p<0.01, ^{∗∗∗}p<0.001. (Panels G and H). Representative histogram plot of p-p38 level in non-cycling (in GO-G1 phase of cell cycle) HSC-enriched populations versus cycling HSC-enriched cells (in S-G₂-M phase of cell cycle, determined by Hoechst 33342 staining). Numbers indicate MFI. Panels I and J: Quantitative fold-change in p-p38 MFI in cycling vs non-cycling (Panel I) and untransduced (GFP) versus transduced (GFP+) HSC enriched cells from 3 independent experiments is shown as mean ± SEM. Statistics: Student's t test, ^{∗}p<0.05. Panel K: Human MPB derived CD34⁺ cells were cultured with or without various p38 inhibitors (B= BIRB-796, Vx= VX 745, and Ly= Ly2228820) and transduced with retrovirus (RV) for 72 hours. Representative histogram plots of p-p38 levels in human CD34⁺ 38⁻ 90⁺ cells are shown. Panel I: Quantitative fold change in p-p38 mean fluorescence intensity (MFI) in human CD34⁺ 38⁻ 90⁺ cells compared to unmanipulated (Panel H) is shown. Data is expressed as mean ± SEM from 5 independent experiments using 5 different MPB donors. Statistics: Student's t test, ^{∗}p<0.05, ^{∗∗}p<0.01, ^{∗∗∗}p<0.001.

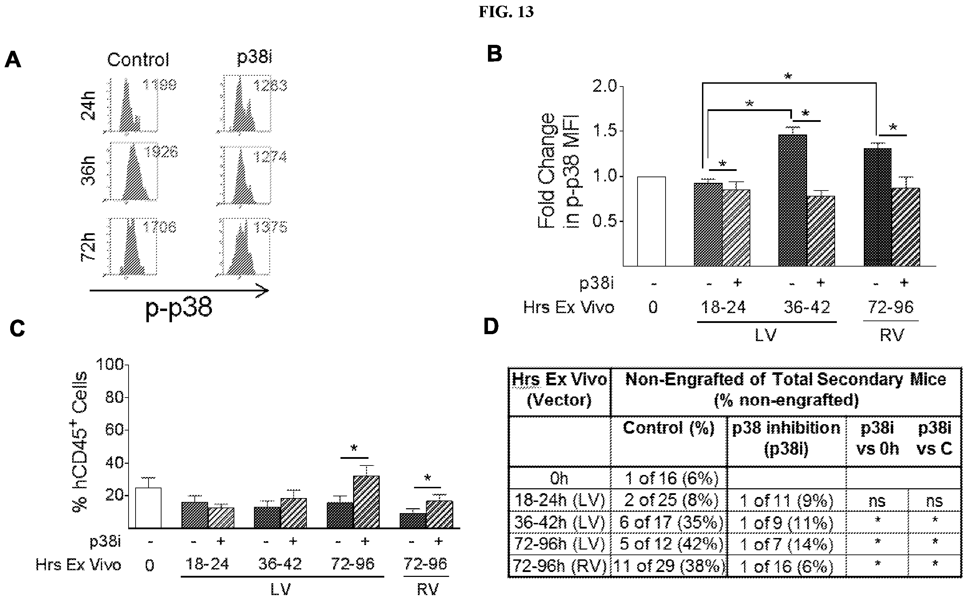

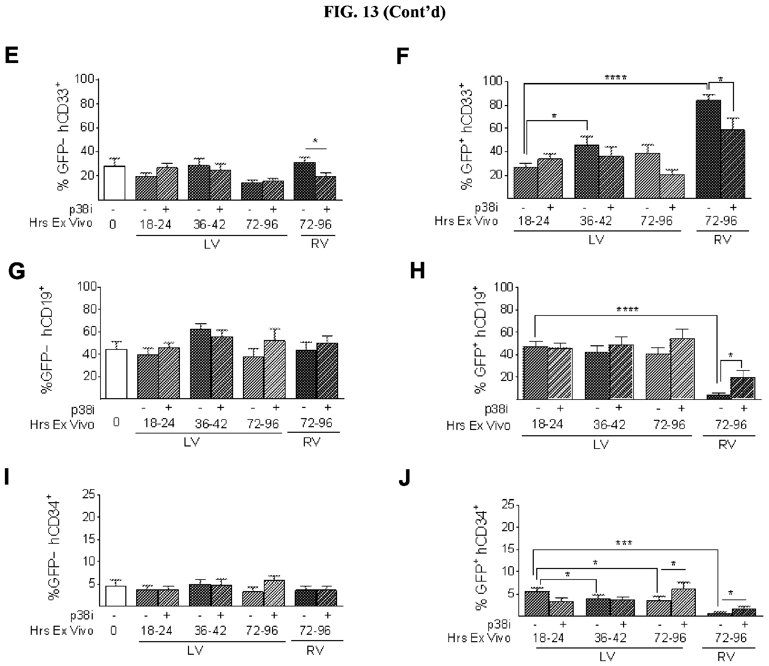

Fig. 13 shows inhibition of p38MAPK during *ex vivo* culture rescues the long term repopulating potential (LTRP) of HSCs and partially reverts the myeloid skewing phenotype. Human MPB derived CD34⁺ cells either fresh or thawed were cultured and transduced with lentivirus (LV) or retrovirus (RV) expressing GFP for the indicated time points. Cells were harvested and phospho-p38MAPK was detected by flow cytometry. Panel A: Representative histogram plot of p-p38 (number represents mean fluorescence intensity (MFI). Panel B: Fold change of p-p38 MAPK MFI (± SEM) in human CD34⁺ 38⁻ 90⁺ 45RA⁻ 49f⁺ cells with or without p38 inhibitor (p38i). Panel C: Human CD45⁺ engraftment in NSG mice with (striped bars) or without p38 inhibitor (solid bars) after 24 weeks of primary transplant. Panel D: Human engraftment in NSG mice with or without p38i after 6 weeks of secondary transplant. Engrafted mice (>0.01*%* CD45⁺ cells in the whole bone marrow) over total mice transplanted is shown as percentage. Human CD33⁺ myeloid (Panels E and F), CD19⁺ B-Lymphoid (Panels G and H) and CD34⁺ hematopoietic stem/progenitor cells (HSPCs) (Panels I and J) re-constitution with or without p38i (Panels E, G, and I): GFP⁻ untransduced; (Panels F, H & J): GFP⁺ transduced in primary transplanted mice 24 weeks post transplantation are shown. Data expressed as mean ± SEM from 5 independent experiments (n=12-21 mice per group). Statistics: Student's t test, ^{∗}p<0.05, ^{∗∗}p<0.01, ^{∗∗∗}p<0.001.

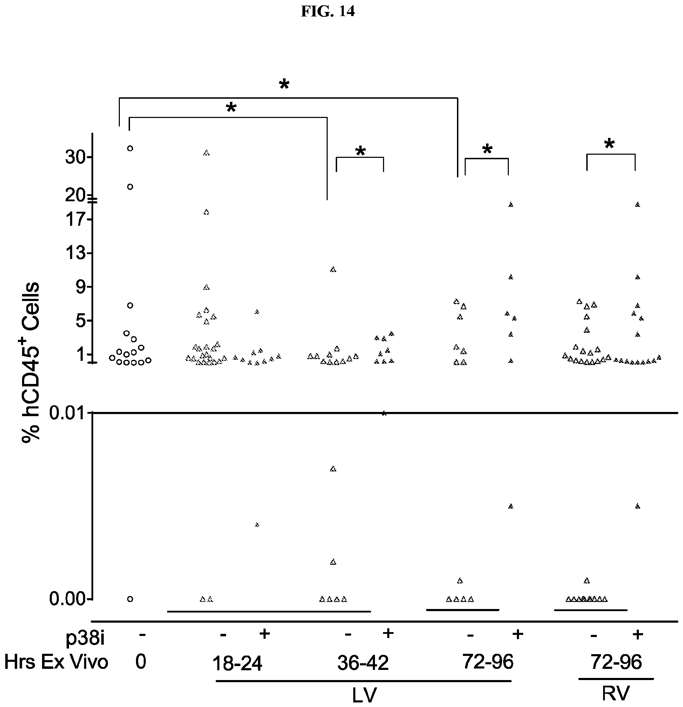

Fig. 14 shows inhibition of p38MAPK during *ex vivo* culture retains the human engraftment in the secondary transplanted NSG mice. Human engraftment in NSG mice with or without BIRB-796 after 6 weeks of secondary transplant. Engrafted mice (>0.01% CD45⁺ cells in the whole bone marrow) are shown above the middle bar, engrafted/Total mice number is shown on the side. Empty triangles are control & filled triangles are BIRB-796 treated. Each symbol represents an individual mouse. Data expressed as mean ± SEM from 5 independent experiments (n=7-29 mice per group). Statistics: Student's t test, ^{∗}p<0.05, ^{∗∗}p<0.01, ^{∗∗∗}p<0.001.

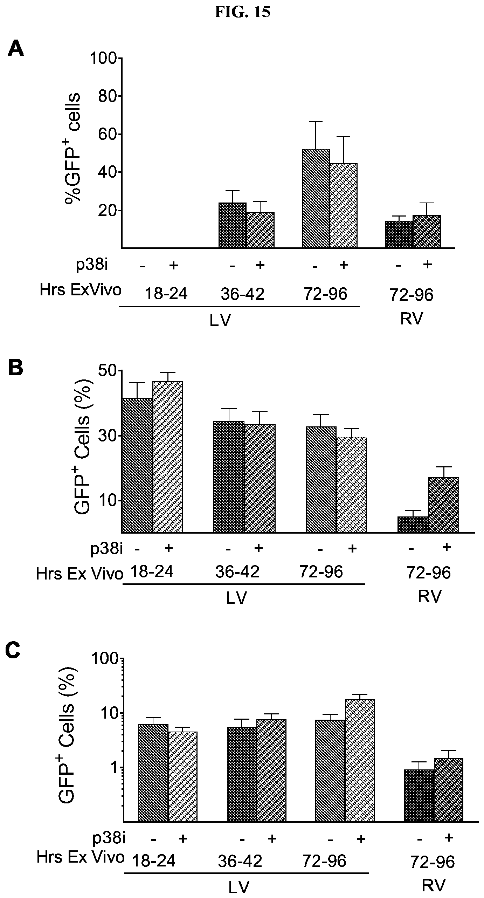

Fig. 15 shows marking in bone marrow of NSG mice. Human CD34⁺ cells were cultured as described in Fig. 13 and transplanted into NSG mice. Total GFP⁺ cells were analyzed *in vitro* before transplant (Panel A) and *in vivo* at 6 weeks (Panel B) and 24 weeks (Panel C) post primary transplant. In Panel A 18-24 hours was not sufficient time for measuring GFP expression and thus not shown. Data expressed as mean ± SEM from 5 independent experiments (n=12-21 mice per group). Statistics: Student's t test, ^{∗}p<0.05, ^{∗∗}p<0.01, ^{∗∗∗}p<0.001.

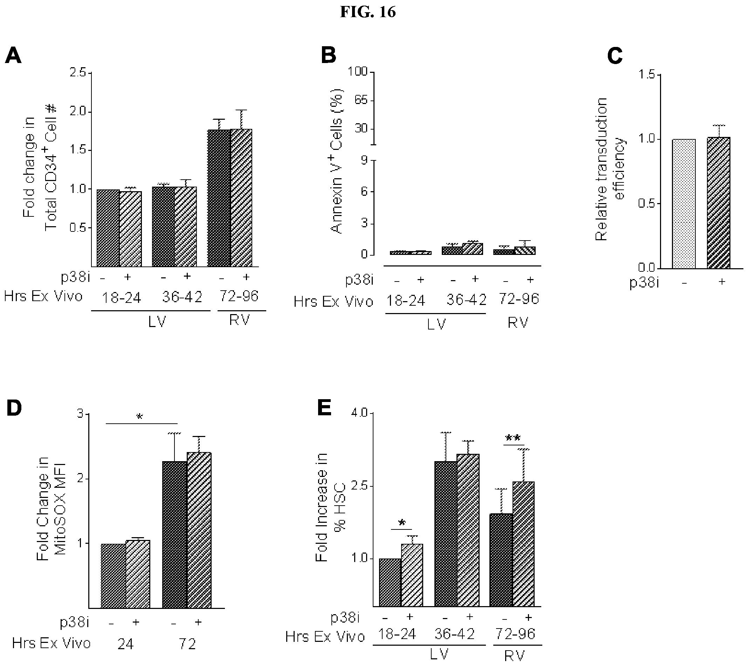

Fig. 16 shows p38 inhibition does not change total CD34⁺ cell number/viability, apoptosis, and ROS level but may retain the percentage of phenotypic HSCs. Human CD34⁺ cells were cultured and transduced as described in Fig. 13, panel A. Harvested cells were stained with trypan blue for viability, total CD34+ cell number (Panel A), fold change in percent human CD34⁺ 38⁻ 90⁺ 45RA⁻ 49f⁺ (HSCs) (Panel B), percent annexin V+ (apoptotic) CD34⁺ 38⁻ 90⁺ cells (Panel C), fold change in transduction efficiency (based on GFP marker percentage) over non-treated CD34⁺ 38⁻ 90⁺ cells (Panel D), fold change in MitoSOX MFI in CD34⁺ 38⁻ 90⁺ cells are shown (Panel E). Data represents mean ± SEM. Statistics: Student's t test, ^{∗∗}0.01>p, ^{∗}p<0.05, ^{∗∗}p<0.01, ^{∗∗∗}p<0.001.

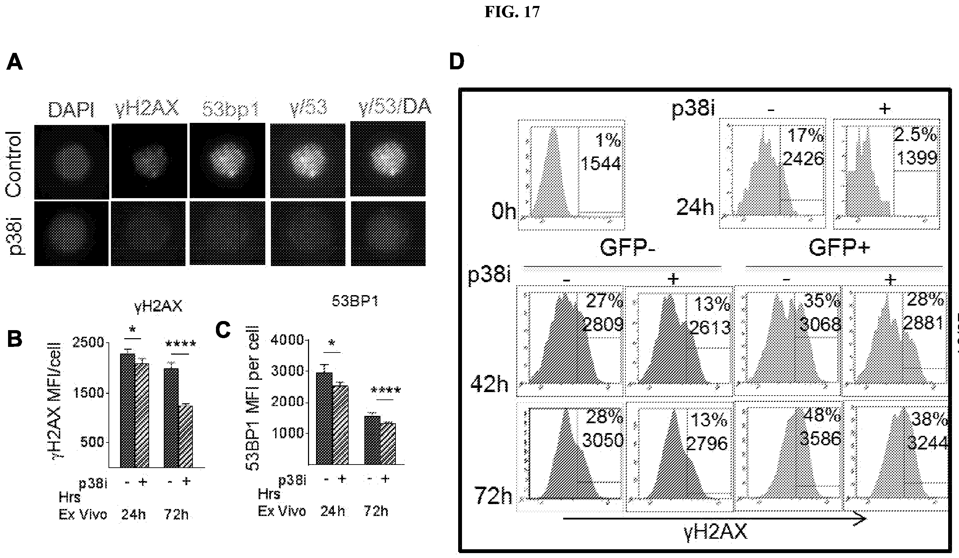

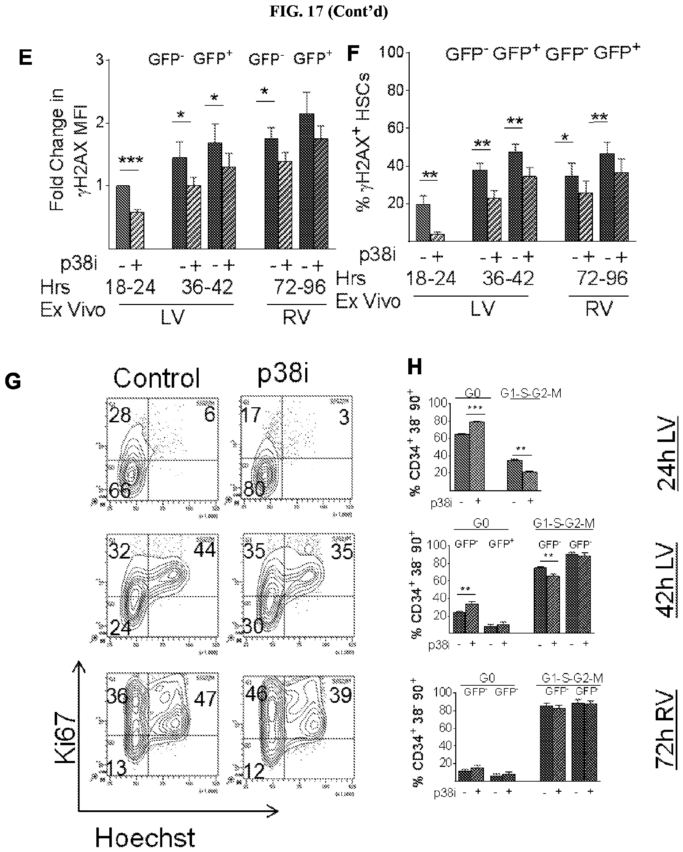

Fig. 17 shows p38MAPK inhibition reduces DNA Damage Response (DDR) and retains HSCs in G₀ quiescent phase during early time period of *ex vivo* culture. Human MPB derived CD34⁺ cells were cultured and transduced with lentivirus (LV) or retrovirus (RV) for the indicated time points. Representative Immunofluorescence images of sorted human CD34⁺ 38⁻ 90⁺ population treated with or without p38i for 72 hours stained with anti-γH2AX and 53BP1 (Panel A) and quantification of γ-H2AX (Panel B) and 53BP1 (Panel C) MFI/ cell. Panel D: Representative histogram plot from flow cytometric analysis of CD34⁺ 38⁻ 90⁺ HSC enriched cells stained for γ-H2AX with or without p38 inhibitor (p38i) and quantification of fold change in mean fluorescence intensity (MFI) of γ-H2AX in HSCs (Panel E) and percent GH2AX⁺ cells (Panel F) at indicated time period. Percentage shown in histograms are cells positive for γH2AX and the numbers below is mean fluorescent intensity (MFI) (n= 5 independent experiments). FACS plot representing HSCs in G₀ (Quiescent), G₁, and S-G₂-M phase of cell cycle with or without p38i (Panel G) and quantification (Panel H) of HSC enriched population at different cell cycle phase at indicated time of *ex vivo* culture. Percentage of (± SEM) Ki67⁻ (G₀) and Ki67⁺ (G1-S-G₂-M) HSCs is depicted on the γ-axis.(n=4 independent experiments for 24h LV and n=5 independent experiments for 42h LV and 72h RV)). Statistics: paired Student's t test, ^{∗}p<0.05, ^{∗∗}p<0.01, ^{∗∗∗}p<0.001.

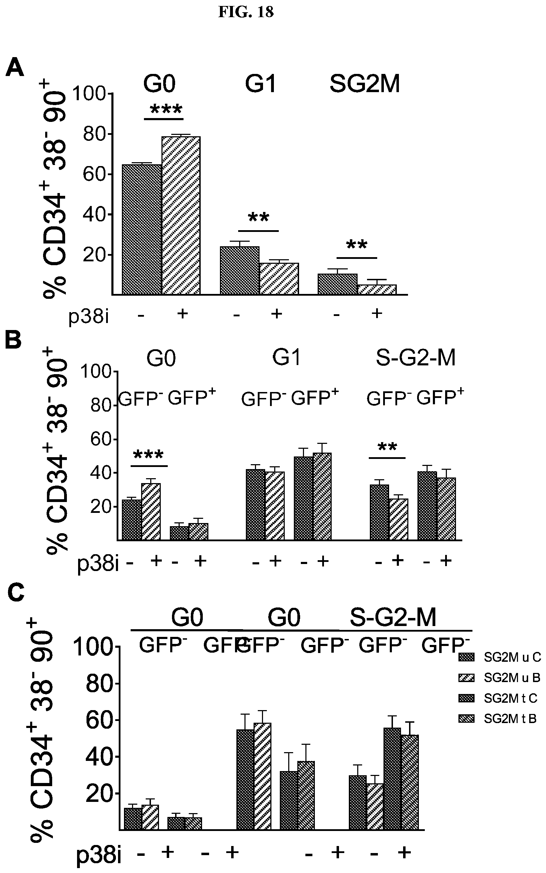

Fig. 18 shows that p38MAPK inhibition reduces DNA Damage Response (DDR) in human CD34⁺ 38⁻ 90⁺ cells during *ex vivo* culture and gene transfer. Human MPB derived CD34⁺cells were cultured and transduced as described in Fig. 17. Panel A: Quantification of γ-H2AX⁺ CD34⁺ 38⁻ 90⁺ cells at 24, 42, & 72 hours. n= 5-8 independent experiments/condition). Panel B: Quantification of immunofluorescence analysis for γ-H2AX (left) and 53BP1 (right) MFI per sorted human CD34⁺ 38⁻ 90⁺ cell with or without p38i for 42 hours. Panel C: Quantitative plot representing CD34⁺ 38⁻ 90⁺ cells in G₀-G₁, and S-G₂-M phase of cell cycle with or without p38i at 42 hours of *ex vivo* culture and lentivirus (LV) transduction. (n=6 independent experiments/condition). Statistics: paired Student's t test, ^{∗}p<0.05, ^{∗∗}p<0.01, ^{∗∗∗}p<0.001. Statistics: Student's t test, ^{∗}p<0.05, ^{∗∗}p<0.01, ^{∗∗∗}p<0.001.

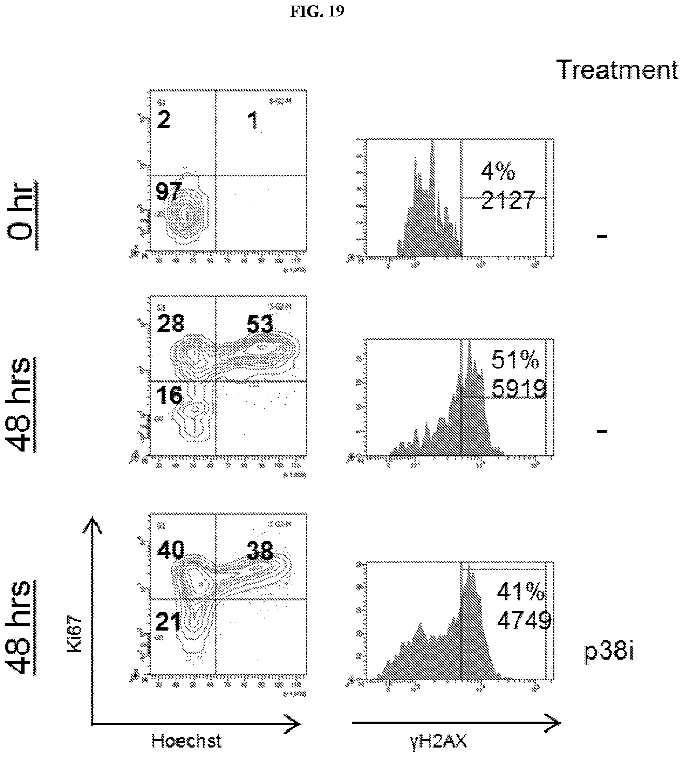

Fig. 19 shows that p38MAPK inhibition also reduces DNA Damage Response (DDR) in human CD34⁺ 38⁻ 90⁺ cells derived from bone marrow (BM). Human BM derived CD34 cells were cultured and transduced with lentivirus (LV) for 48 hours. Representative FACS plot representing bone marrow derived HSC enriched CD34⁺ 38⁻ 90⁺ population in G₀ (Quiescent), G₁, and S-G₂-M phase of cell cycle with or without p38i (left) and the corresponding representative histogram plot of γH2AX at indicated time and treatment. Percentage shown in histogram refers to cells positive for γH2AX and the numbers below is mean fluorescent intensity (MFI).

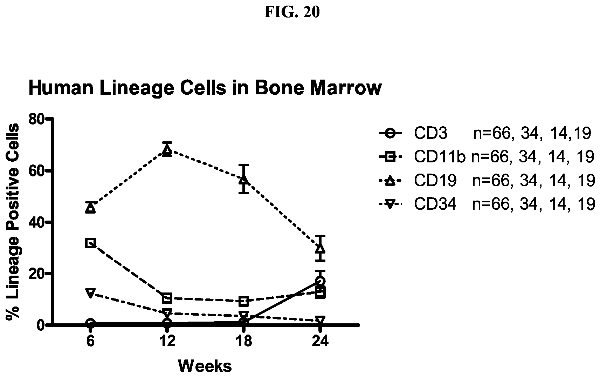

Fig. 20 shows human lineage cells in bone marrow over time. Nearly a tenfold higher CD34⁺ cell dose is needed for robust engraftment. Multilineage engraftment kinetics are much slower. Secondary transplants require long-term multilineage engraftment. Engraftment in blood does not reflect engraftment in bone marrow.

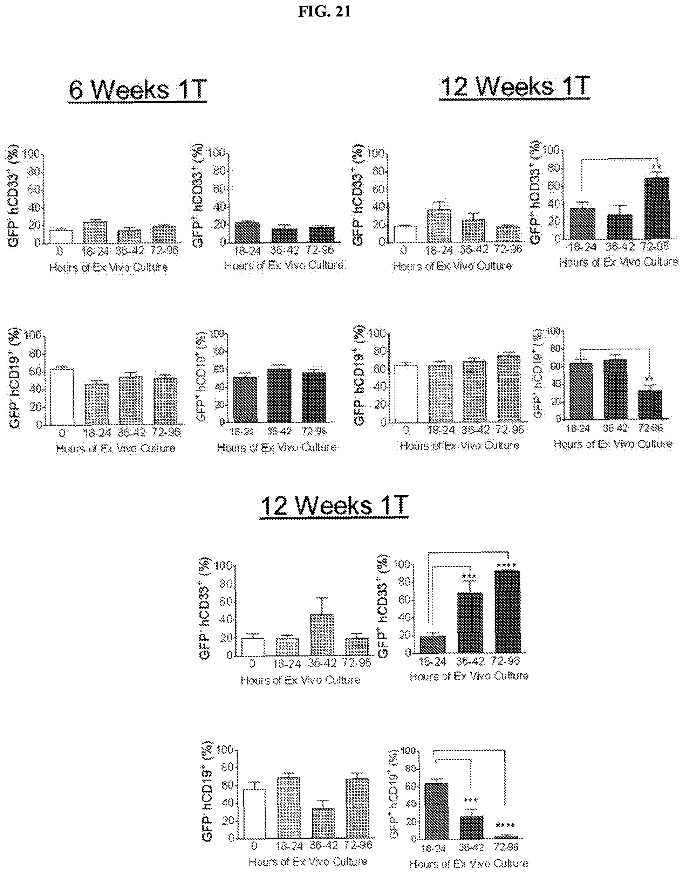

Fig. 21 shows that hHSC progeny becomes myeloid biased with increased time in culture.

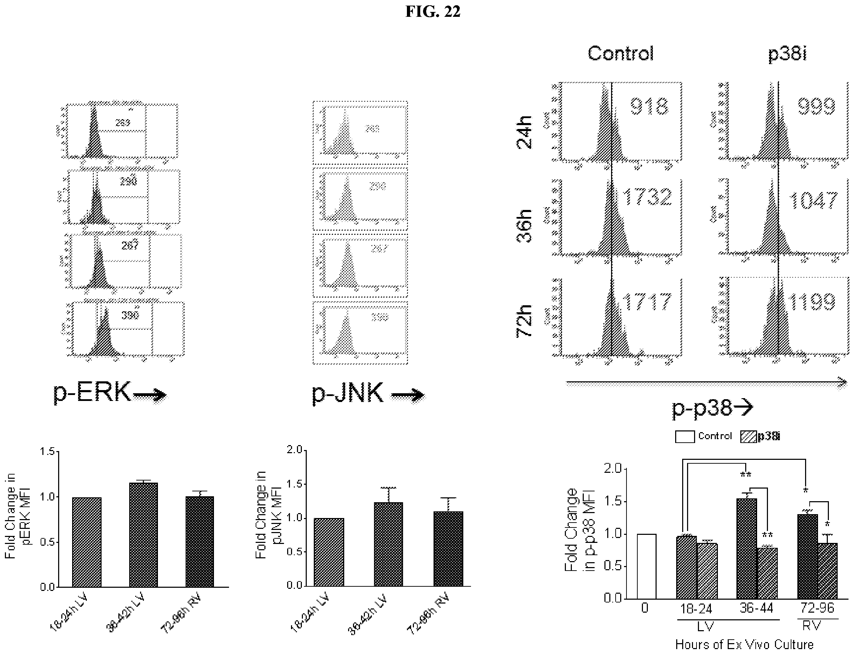

Fig. 22 shows that *ex vivo* manipulation increases HSC stress signaling.

### DETAILED DESCRIPTION OF THE INVENTION

Hematopoietic stem cells (HSCs) are desirable targets for gene therapy for various inherited hematological diseases including, *e*.*g*., hemoglobinopathies. However, gene transfer into HSCs typically involves *ex vivo* manipulation and culture, which results in a large amount of HSC loss with their differentiation to hematopoietic progenitor cells (HPCs), making these cells less competent at engraftment *in vivo.* Currently, the limited number of HSCs that repopulate after autologous transplant is a major limitation to effective gene transfer. Thus, large transduced HSCs at myelo-ablative conditioning to destroy resident HSCs are currently required to provide an engraftment advantage. However, the source of HSCs is limited and myelo-ablative conditioning could induce adverse effects or complications in patients who are sensitive to such procedure. Accordingly, there is a need to develop methods and compositions for preparing hematopoietic stem cells (HSCs) for increased engraftment in subjects who are in need of a HSC transplantation.

The present disclosure is based, at least in part, on the unexpected discovery that extended *ex vivo* culture and gene manipulation induce a DNA damage response (DDR) in hematopoietic stem cells (HSCs) during *ex vivo* culture, which is mediated by activation of p38 MAPK stress signaling. For example, such HSCs after extended *ex vivo* culture and gene manipulation showed increased DNA double strand breaks (for example, increased γ-H2AX signaling), increased DNA damage response (for example, increased 53BP1 signaling), loss of long term repopulating potential, increased myeloid lineage bias, and/or increased phosphorylation level of p38 MAPK in the HSCs. Further, it was discovered that blocking the p38 MAPK signaling (*e*.*g*., via an anti-p38 MAPK inhibitor) significantly decreased DNA double strand breaks and/or DNA damage response that occurred in HSCs with both increasing time in culture and with transduction, retained long germ repopulating potential, reduced myeloid lineage skewing in the HSCs, and/or increased engraftment of HSCs transplanted *in vivo.* Thus, p38 MAPK inhibitors would be expected to enhance the engraftment capacity of HSCs cultured *ex vivo via* blocking the p38 MAPK stress signaling and reducing, *e.g.,* DNA double strand breaks.

Accordingly, in some aspects, the present disclosure provides *ex vivo* cell culture methods for preserving the stemness of stem cells such as HSCs in the presence of one or more p38 MAPK inhibitors, which suppresses at least DNA damage response due to extended *ex vivo* culture and genetic manipulation. Such stem cells may have undergone a manipulation that causes a DNA double-strand break, for example, transduced by a vector that is capable of integrating into the genome of the stem cells, or induced by genome editing. Genome editing methods are generally classified based on the type of endonuclease that is involved in generating double stranded breaks in the target nucleic acid. Examples of genome editing methods include, e.g., use of zinc finger nucleases (ZFN), transcription activator-like effector-based nuclease (TALEN), meganucleases, and/or CRISPR/Cas systems. Without wishing to be bound by any particular theory, *ex vivo* manipulation of HSCs may activate stress signaling, resulting in their commitment to hematopoietic progenitor cells (HPCs) at the expense of HSC self-renewal. Events that involve DNA double-strand breaks, *(e.g.,* vector integration events) may exacerbate HSC loss and the present discovery showed that blocking the p38 MAPK signaling pathway would rescue HSC loss in *ex vivo* culturing. Accordingly, described herein are also *ex vivo* methods and compositions for preparing stem cells such as HSCs having enhanced engraftment activity using one or more p38 MAPK inhibitors. The methods and compositions described herein promote engraftment of HSCs in a subject (e.g., a human patient) after HSC transplantation.

### I. p38 Mitogen-Activated Protein Kinases (MAPK) Inhibitors

p38 mitogen-activated protein kinases (MAPKs) are a class of mitogen-activated protein kinases that are responsive to stress stimuli, such as cytokines, ultraviolet irradiation, heat shock, and/or osmotic shock. The p38 MAPK family includes four members, p38-α (MAPK14), p38-β (MAPK11), p38-γ (MAPK12 / ERK6), and p38-δ (MAPK13 / SAPK4), which are involved in a signaling cascade that controls cellular response to cytokine and stress. Inhibitors for any of the p38 MAPK members can be used in the *ex vivo* culturing methods described herein. In some examples, the inhibitors used herein are specific to one of the members, for example, specific to p38-α, p38-β, p38-γ, or p38-δ. In other examples, the p38 MAPK inhibitors are universal to two or more members of the p38 MAPK family. In one example, the inhibitors used herein are specific or selective to p38-α (MAPK14).

As used herein, the term "p38 MAPK" refers to a p38 MAPK polypeptide having the same or similar bioactivity of a wild-type p38 MAPK. Ap38 MAPK polypeptide may have an amino acid sequence that is at least 70% or more (including at least 75%, at least 80%, at least 85%, at least 90%, at least 95%, at least 97%, at least 99%, or 100%) identical to that of a wild-type p38 MAPK, and is capable of trigger p38α signaling pathway.

The "percent identity" of two amino acid sequences is determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST protein searches can be performed with the XBLAST program, score=50, wordlength=3 to obtain amino acid sequences homologous to the protein molecules as described herein. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (*e.g.*, XBLAST and NBLAST) can be used.

Wild-type p38 MAPK sequences (*e.g.*, sequences of p38-α (MAPK14), p38-β (MAPK11), p38-γ (MAPK12 / ERK6), and p38-δ (MAPK13 / SAPK4)) of various species are available on the world wide web from the NCBI, including human, mouse, and rat. For example, the nucleotide sequence encoding an isoform of human p38- α (MAPK14) is available at NCBI under Accession No. NM_001315 and its corresponding amino acid sequence is under Accession No. NP_001306.

As used herein, the term "p38 MAPK inhibitor" refers to a molecule that partially or fully blocks, inhibits, or neutralizes a biological activity of a p38 MAPK protein. Suitable inhibitor molecules specifically include antagonist antibodies or antibody fragments, fragments or amino acid sequence variants of native polypeptides, peptides, antisense oligonucleotides, small organic molecules, recombinant proteins or peptides, etc. Methods for identifying inhibitors of a polypeptide can comprise contacting a polypeptide with a candidate p38 MAPK inhibitor molecule and measuring a detectable change in one or more biological activities normally associated with the polypeptide.

A p38 MAPK inhibitor can be a molecule of any type that interferes with the signaling associated with at least one or more p38 MAPK family members (*e*.*g*., p38-α (MAPK14), p38-β (MAPK11), p38-γ (MAPK12 / ERK6), and p38-δ (MAPK13 / SAPK4)) in a cell, for example, either by decreasing transcription or translation of p38 MAPK-encoding nucleic acid, or by inhibiting or blocking p38 MAPK polypeptide activity, or both. In some examples, a p38 MAPK inhibitor is an agent that interferes with the signaling associated with p38-α (MAPK). Examples of p38 MAPK inhibitors include, but are not limited to, antisense polynucleotides, interfering RNAs, catalytic RNAs, RNA-DNA chimeras, p38 MAPKspecific aptamers, anti-p38 MAPK antibodies, p38 MAPK-binding fragments of anti-p38 MAPK antibodies, p38 MAPK-binding small molecules, p38 MAPK-binding peptides, and other polypeptides that specifically bind p38 MAPK (including, but not limited to, p38 MAPK-binding fragments of one or more p38 MAPK ligands, optionally fused to one or more additional domains), such that the interaction between the p38 MAPK inhibitor and p38 MAPK results in a reduction or cessation of P38 MAPK activity or expression. It will be understood by one of ordinary skill in the art that in some instances, a p38 MAPK inhibitor can antagonize or neutralize one p38 MAPK activity without affecting another p38 MAPK activity. For example, a desirable p38 MAPK inhibitor for use in certain of the methods herein is a p38 MAPK inhibitor that binds p38-α and blocks p38 MAPK signaling, *e*.*g*., without affecting or minimally affecting any of the other p38 MAPK interactions, for example, binding p38-β, p38-γ, and/or p38-δ.

In some embodiments, p38 MAPK inhibitors used for the methods described herein are cell-permeable.

In some embodiments, a p38 MAPK inhibitor is an agent that directly or indirectly inhibits or reduces DNA double strand breaks and/or DNA damage response in genetically manipulated stem cells such as HSCs *(e.g.,* by using an integrating vector such as viral vectors or performing genome editing, e.g., which involves use of zinc finger nucleases (ZFN), transcription activator-like effector-based nuclease (TALEN), meganucleases, and/or CRISPR/Cas systems), wherein the DNA double strand breaks and/or DNA damage response are mediated by one or more family members of p38 MAPK (*e.g.,* p38-α, p38-β, p38-γ, p38-δ, and any combinations thereof). Accordingly, a p38 MAPK inhibitor can target the p38 MAPK (*e.g.,* p38-α, p38-β, p38-γ, p38-δ, and any combinations thereof) or any of p38 MAPK's upstream molecules. Examples of p38 MAPK inhibitors include, without limitations, anti-p38-α molecules, anti-p38-β molecules, anti-p38-y molecules, anti-p38-δ molecules, and any combinations thereof. A p38 MAPK inhibitor can be a protein, a peptide, a peptidomimetic, an aptamer, a nucleic acid, an antibody, a small molecule, or any combinations thereof.

A p38 MAPK inhibitor can be a molecule *(e.g.,* an antibody, an aptamer, or a small molecule) that interferes with the binding of one or more family members of p38 MAPK (*e.g.,* p38-α, p38-β, p38-γ, p38-δ, and any combinations thereof). Alternatively, the p38 MAPK inhibitor can be a molecule (*e.g.,* a inhibitory polynucleotide or oligonucleotide such as interfering RNA or antisense oligonucleotide) that suppresses transcription and/or translation of one or more family members of p38 MAPK (*e.g.,* p38-α, p38-β, p38-γ, p38-δ, and any combinations thereof), thereby reducing the mRNA/protein level of this enzyme. The p38 MAPK inhibitor as described herein may reduce the P38 MAPK signaling in stem cells or HSCs *(e.g.,* during *ex vivo* culture after genetic manipulation) by at least 20% or more, *e.g.,* 30%, 40%, 50%, 60%, 70%, 80%, 90%, 95% or above. The inhibitory activity of such an inhibitor against p38 MAPK can be determined by conventional methods, *e*.*g*., measuring the phosphorylation level of p-p38, for example, using protein assays such as ELISA or Western blot.

In some embodiments, the p38 MAPK inhibitor is an antibody that specifically binds to one or more family members of p38 MAPK (*e.g.,* p38-α, p38-β, p38-γ, p38-δ, and any combinations thereof) and neutralizes its activity to activate p38 MAPK signaling pathway. As used herein, the term "antibody" as includes but is not limited to polyclonal, monoclonal, humanized, chimeric, Fab fragments, Fv fragments, F(ab') fragments and F(ab')2 fragments, as well as single chain antibodies (scFv), fusion proteins and other synthetic proteins which comprise the antigen-binding site of the antibody.

Antibodies can be made by the skilled person using methods and commercially available services and kits known in the art. Methods of preparation of monoclonal antibodies are well known in the art and include hybridoma technology and phage display technology. Further antibodies suitable for use in the present disclosure are described, for example, in the following publications: Antibodies A Laboratory Manual, Second edition. Edward A. Greenfield. Cold Spring Harbor Laboratory Press (September 30, 2013); Making and Using Antibodies: A Practical Handbook, Second Edition. Eds. Gary C. Howard and Matthew R. Kaser. CRC Press (July 29, 2013); Antibody Engineering: Methods and Protocols, Second Edition (Methods in Molecular Biology). Patrick Chames. Humana Press (August 21, 2012); Monoclonal Antibodies: Methods and Protocols (Methods in Molecular Biology). Eds. Vincent Ossipow and Nicolas Fischer. Humana Press (February 12, 2014); and Human Monoclonal Antibodies: Methods and Protocols (Methods in Molecular Biology). Michael Steinitz. Humana Press (September 30, 2013)).

Antibodies may be produced by standard techniques, for example by immunization with the appropriate polypeptide or portion(s) thereof, or by using a phage display library. If polyclonal antibodies are desired, a selected mammal (*e*.*g*., mouse, rabbit, goat, horse, etc) is immunized with an immunogenic polypeptide bearing a desired epitope(s), optionally haptenized to another polypeptide. Depending on the host species, various adjuvants may be used to increase immunological response. Such adjuvants include, but are not limited to, Freund's, mineral gels such as aluminum hydroxide, and surface active substances such as lysolecithin, pluronic polyols, polyanions, peptides, oil emulsions, keyhole limpet hemocyanin, and dinitrophenol. Serum from the immunized animal is collected and treated according to known procedures. If serum containing polyclonal antibodies to the desired epitope contains antibodies to other antigens, the polyclonal antibodies can be purified by immunoaffinity chromatography or any other method known in the art. Techniques for producing and processing polyclonal antisera are well known in the art.

A p38 MAPK inhibitor specifically binds to one member of p38 MAPK (*e.g.,* p38-α, p38-β, p38-γ, or p38-δ) if the inhibitor binds to the specific member of p38 MAPK with a greater affinity than for an irrelevant polypeptide. In some embodiments, the inhibitor binds to one member of p38 MAPK (*e.g.,* p38-α, p38-β, p38-γ, or p38-δ) with at least 5, or at least 10 or at least 50 times greater affinity than for the irrelevant polypeptide. In some embodiments, the inhibitor binds to one member of p38 MAPK (*e.g.,* p38-α, p38-β, p38-γ, or p38-δ) with at least 100, or at least 1,000, or at least 10,000 times greater affinity than for the irrelevant polypeptide. Such binding may be determined by methods well known in the art, such surface plasmon resonance such as a Biacore^{®} system. In some embodiments, the inhibitor has an affinity (as measured by a dissociation constant, K_{D}) for a specific member of p38 MAPK (*e.g.,* p38-α, p38-β, p38-γ, or p38-δ) of at least 10⁻⁷ M, 10⁻⁸ M, 10⁻⁹ M, 10⁻¹⁰ M, or 10⁻¹¹ M.

In some embodiments, the p38 MAPK inhibitor is a small molecule, such as a small organic molecule, which typically has a molecular weight less than 5,000 kDa. Suitable small molecules include those that bind to one or more family members of p38 MAPK (*e.g.,* p38-α, p38-β, p38-γ, or p38-δ) or a fragment thereof, and may be identified by methods such as screening large libraries of compounds (Beck- Sickinger & Weber (2001) Combinational Strategies in Biology and Chemistry (John Wiley & Sons, Chichester, Sussex); by structureactivity relationship by nuclear magnetic resonance (Shuker et al (1996) "Discovering highaffinity ligands for proteins: SAR by NMR. Science 274: 1531-1534); encoded self-assembling chemical libraries Melkko et al (2004) "Encoded self-assembling chemical libraries." Nature Biotechnol. 22: 568-574); DNA-templated chemistry (Gartner et al (2004) "DNA-tem plated organic synthesis and selection of a library of macrocycles. Science 305: 1601-1605); dynamic combinatorial chemistry (Ramstrom & Lehn (2002) "Drug discovery by dynamic combinatorial libraries." Nature Rev. Drug Discov. 1 : 26-36); tethering (Arkin & Wells (2004) "Small-molecule inhibitors of protein-protein interactions: progressing towards the dream. Nature Rev. Drug Discov. 3: 301-317); and speed screen (Muckenschnabel et al (2004) "SpeedScreen: label-free liquid chromatography-mass spectrometry-based high- throughput screening for the discovery of orphan protein ligands." Anal. Biochem. 324: 241-249). Typically, small molecules will have a dissociation constant for P38 MAPK in the nanomolar range.

Examples of small molecule p38 MAPK inhibitors for use in the *ex vivo* culturing method described herein are provided in Table 1 below:

**Table 1. Exemplary p38 MAPK Inhibitors**

| **Type** | **Inhibitor** | **Chemical Name** |
|---|---|---|
| *Prototypical pyridinyl imidazoles* | SB203580 | 4-[5-(4-Fluorophenyl)-2-[4-(methylsulfonyl)phenyl]-1H-imidazol-4-yl]pyridine |
| | SKF-86002 | 6-(4-Fluorophenyl)-2,3-dihydro-5-(4-pyridinyl)imidazo[2,1-b]thiazole dihvdrochloride |
| *Aryl-pyridyl heterocycles* | SB-242235 | 1-(4-piperidinyl)-4-(4-fluorophenyl)-5-(2-methoxy-4-pyrimidinyl) imidazole |
| | RWJ-67657 | 4-[4-(4-Fluorophenyl)-1-(3-phenylpropyl)-5-(4-pyridinyl)-1H-imidazol-2-yl]-3-butyn-1-ol |
| | SB 239065 | Not Available |
| *Non-aryl-pyridyl heterocycles* | RO3201195 | S-[5-amino-1-(4-fluorophenyl)-1H-pyrazol-4-yl]-[3-(2,3-dihydroxypropoxy)phenyl]methanone |
| | BIRB-796 | 1-[5-tert-butyl-2-(4-methylphenyl)pyrazol-3-yl]-3-[4-(2-morpholin-4-ylethoxy)naphthalen-1-yl]urea), |
| | VX-745 | 5-(2,6-dichlorophenyl)-2-(2,4-difluorophenylthio)-6H-pyrimido[1,6-b]pyridazin-6-one |
| *Other* | SB202190 | 4-[4-(4-fluorophenyl)-5-pyridin-4-yl-1,3-dihydroimidazol-2-ylidene]cyclohexa-2,5-dien-1-one, VX-702 6-(N-carbamoyl-2,6-difluoroanilino)-2-(2,4-difluorophenyl)pyridine-3-carboxamide |
| | LY2228820 | 5-[2-tert-butyl-4-(4-fluorophenyl)-1H-imidazol-5-yl]-3-(2,2-dimethylpropyl)imidazo[4,5-b]pyridin-2-amine; methanesulfonic acid |
| | PH-797804 | 3-[3-bromo-4-[(2,4-difluorophenyl)methoxy]-6-methyl-2-oxopyridin-1-yl]-N,4-dimethylbenzamide |
| | VX-702 | 6-(N-carbamoyl-2,6-difluoroanilino)-2-(2,4-difluorophenyl)pyridine-3-carboxamide) |
| | LY2228820 | 5-[2-tert-butyl-4-(4-fluorophenyl)-1H-imidazol-5-yl]-3-(2,2-dimethylpropyl)imidazo[4,5-b]pyridin-2-amine;methanesulfonic acid |
| | L-167307 (Selective imidazole) | 4-[2-(4-Fluorophenyl)-5-[4-(methylsulfinyl)phenyl]-1Hpyrrol-3-yl]pyridine |
| | Pyridinyloxazole inhibitor | Not Available |
| | RPR-200765A | ((2r,5r)-2-(4-(4-fluorophenyl)-5-(pyridin-4-yl)-1H-imidazol-2-yl)-5-methyl-1,3-dioxan-5-yl)(morpholino)methanone methane sulfonate |
| | RPR-238677 | Not Available |
| | FR167653 | 1-(7-(4-fluorophenyl)-1,2,3,4-tetrahydro-8-(4-pyridyl)pyrazolo(5,1-c)(1,2,4)triazin-2-yl)-2-phenylethanedione sulphate monohydrate |
| | SB-239063 | trans-1-(4-hydroxycyclohexyl)-4-(4-fluorophenyl)-5-(2-methoxypyridimidin-4-yl)imidazole |

Exemplary p38 MAPK inhibitors also include doramapimod (*e*.*g*., BIRB-796), ralimetinib (*e*.*g*., LY2228820 dimesylate), aminopyridine-based, ATP-competitive inhibitors of p38 MAPK *(e.g.,* Vx702), pyridinyl imidazole inhibitors (*e.g.,* SB203580), and any combinations thereof.

Other p38 MAPK inhibitors are well known in the art, for example, those described in U.S. Pat. Nos. 7,169,779, 6,635,644, 6,608,060, 6,632,945, 6,528,508, 6,509,363 (Heterocyclic inhibitors of p38), 6,147,080, 6,800,626, 6,093,742, 6,949,560 (Imidazosubstituted compounds), 6,852,740 (Pyrazole derivatives), 6,630,485, 6,759,410 (3,4-dihydro-(1h)-quinazolin-2ones), 6,696,471 (Aminopyrrole compounds), 6,696,443 (Piperidine/piperazine-type inhibitors), 6,509,361 (1,5-diaryl substituted pyrazoles), 6,444,696 (pyrazole derivatives), and PCT patent publications WO2000017175, WO2000017204, WO1996021654, WO1999000357, WO1999064400. Other p38 MAPK inhibitors as described in Xing "Clinical candidates of small molecule p38 MAPK inhibitors for inflammatory diseases" (2015) MAP Kinase 4: 5508 may also be used for the *ex vivo* methods and compositions described herein.

In some embodiments, the p38 MAPK inhibitor is an interfering RNA such as a small interfering RNA (siRNA) short hairpin RNA (shRNA). In some embodiments, the p38 MAPK inhibitor is a small interfering RNA (siRNA) that binds to the mRNA of one or more family members of p38 MAPK (*e.g.,* p38-α, p38-β, p38-γ, or p38-δ) and blocks its translation or degrades the mRNA via RNA interference. Exemplary small interfering RNAs are described by Hannon et al. Nature, 418 (6894): 244-51 (2002); Brummelkamp et al., Science 21 , 21 (2002); and Sui et al., Proc. Natl Acad. Sci. USA 99, 5515-5520 (2002). RNA interference (RNAi) is the process of sequence- specific post-transcriptional gene silencing in animals initiated by double-stranded (dsRNA) that is homologous in sequence to the silenced gene. siRNAs are generally RNA duplexes with each strand being 20-25 (such as 19-21) base pairs in length. In some embodiments, the p38 MAPK inhibitor is a short hairpin RNA (shRNA) that is complementary to a p38 MAPK nucleic acid *(e.g.,* a p38 MAPK mRNA). An shRNA typically contains of a stem of 19-29 base pairs, a loop of at least 4 nucleotides (nt), and optionally a dinucleotide overhang at the 3' end. Expression of shRNA in a subject can be obtained by delivery of a vector (*e.g.,* a plasmid or viral or bacterial vectors) encoding the shRNA. siRNAs and shRNAs may be designed using any method known in the art or commercially available (see, *e.g.,* products available from Dharmacon and Life Technologies). An siRNA may also comprise one or more chemical modifications, such as a base modification and/or a bond modification to at least improve its stability and binding affinity to the target mRNA.

In some embodiments, the p38 MAPK inhibitor is an antisense oligonucleotide that is complementary to a p38 MAPK nucleic acid *(e.g.,* a p38 MAPK mRNA). Antisense oligonucleotides are generally single-stranded nucleic acids (either a DNA, RNA, or hybrid RNA-DNA molecule), which are complementary to a target nucleic acid sequence, such as a portion of a p38 MAPK mRNA. By binding to the target sequence, an RNA-RNA, a DNA-DNA, or RNA-DNA duplex is formed, thereby inhibiting the function or level of the target nucleic acid, such as by blocking the transcription, processing, poly(A) addition, replication, translation, or promoting inhibitory mechanisms of the cells, such as promoting mRNA degradation. In some embodiments, an antisense oligonucleotide is 10 to 40, 12 to 35, or 15 to 35 bases in length, or any integer in between. An antisense oligonucleotide can comprise one or more modified bases, such as 2-Aminopurine, 2,6-Diaminopurine (2-Amino-dA), 5-Bromo dU, 5-Methyl dC, deoxyInosine, Locked Nucleic Acid (LNA), 5-Nitroindole, 2'-O-Methyl bases, Hydroxmethyl dC, 2' Fluoro bases. An antisense oligonucleotide can comprise one or more modified bonds, such as a phosphorothioate bond.

In some embodiments, the p38 MAPK inhibitor is a ribozyme that is complementary to a p38 MAPK nucleic acid *(e.g.,* a p38 MAPK mRNA) and cleaves the p38 MAPK nucleic acid. Ribozymes are RNA or RNA-protein complexes that cleave nucleic acids in a sitespecific fashion. Ribozymes have specific catalytic domains that possess endonuclease activity. The ribozymes of the present disclosure may be synthetic ribozymes, such as those described in U.S. Pat. No. 5,254,678. These synthetic ribozymes have separate hybridizing regions and catalytic regions; therefore, the hybridizing regions can be designed to recognize a target sequence, such as a p38 MAPK sequence.

siRNAs, shRNAs, ribozymes, and antisense oligonucleotides as described herein may be complementary to a p38 MAPK nucleic acid *(e.g.,* a p38 MAPK mRNA), or a portion thereof. It is to be understood that complementarity includes 100% complementarity but does not necessarily exclude mismatches at one or more locations, resulting in, *e.g.,* at least 80%, at least 90%, at least 95%, at least 98%, or at least 99% complementarity.

In some embodiments, the p38 MAPK inhibitor is a non-antibody peptide or protein. The peptide or protein may comprise an amino acid sequence that interferes with the p38 MAPK signaling. Proteins and peptides may be designed using any method known in the art, *e.g.,* by screening libraries of proteins or peptides for binding to p38 MAPK or inhibition of p38 MAPK binding to a ligand, such as p38 MAPK.

The capability of a candidate compound, such as a small molecule, protein, or peptide, to bind to or interact with a p38 MAPK polypeptide or fragment thereof may be measured by any method of detecting/measuring a protein/protein interaction or other compound/protein interaction. Suitable methods include methods such as, for example, yeast two-hybrid interactions, co-purification, ELISA, co-immunoprecipitation and surface plasmon resonance methods. Thus, the candidate compound may be considered capable of binding to the polypeptide or fragment thereof if an interaction may be detected between the candidate compound and the polypeptide or fragment thereof by ELISA, co-immunoprecipitation or surface plasmon resonance methods or by a yeast two-hybrid interaction or co-purification method, all of which are known in the art. Screening assays which are capable of high throughput operation are also contemplated. Examples may include cell based assays and protein-protein binding assays.

### II. Methods for Preserving Stemness of Stem Cells in Ex Vivo Culture

Any of the p38 MAPK inhibitors, *e*.*g*., those described herein, can be used for preserving stemness of stem cells (*e.g.*, hematopoietic stem cells) in *ex vivo* or *in vitro* culture. Stemness refers to the ability of unspecialized cells to renew themselves as unspecialized cells but still retain this ability to specialize to produce specific types of cells. The stem cell potential, or "stemness" of stem cells (*e*.*g*., hematopoietic stem cells) relies upon a combination of properties: quiescence, repopulation potential, self-renewal potential, and multi-lineage differentiation potential. Cell-cycle quiescence in stem cells *(e.g.,* HSCs) maintains stemness by protecting cells from differentiation or senescence.

The present disclosure features *ex vivo* culturing methods for preserving the stemness of stem cells in cell cultures by culturing stem cells (*e.g.*, HSCs) in the presence of one or more p38 MAPK inhibitors. The stem cell thus prepared can be used in treating suitable diseases *via* stem cell transplantation.

To perform the *ex vivo* culturing methods described herein, a suitable population of stem cells *(e.g.,* pluripotent stem cells) can be obtained from a suitable source. In some instances the population of stem cells *(e.g.,* HSCs) can be derived from a human subject, *e.g.,* from the bone marrow cells, peripheral blood cells, and/or umbilical cord blood cells of the human subject, *via* a convention method. In some examples, the stem cells are adult stem cells *(e.g.,* HSCs), which can be derived from the bone marrow or peripheral blood cells of a human adult. In some examples, the stem cell population is substantially free of umbilical stem cells.

In some embodiments, any of the stem cell populations described herein have undergone a genetic manipulation that causes a DNA damage, *e*.*g*., double strand breaks, dimerization or cross-linking, unpaired bases, modified bases, conversion of one base into another resulting in unpaired bases, chromatin unwinding or other modifications, etc. In some embodiments, any of the stem cell populations described herein have undergone a genetic manipulation that a double strand break. A genetic manipulation includes modifying, inserting, or deleting at least one of the genes in the stem cells *(e.g.,* HSCs). Genetic manipulation may include transduction with a vector that is capable of being integrated into the cell genome.

A "vector", as used herein is any nucleic acid vehicle (DNA or RNA) capable of facilitating the transfer of a nucleic acid molecule into stem cells *(e.g.,* HSCs). In general, vectors include, but are not limited to, plasmids, phagemids, viral vectors, and other vehicles derived from viral or bacterial sources that have been manipulated by the insertion or incorporation of a target nucleotide sequence. Viral vectors include, but are not limited to vectors comprising nucleotide sequences derived from the genome of the following viruses: retrovirus; lentivirus; adenovirus; adeno-associated virus; SV40-type viruses; polyoma viruses; Epstein-Barr viruses; papilloma viruses; herpes virus; vaccinia virus; polio virus. One can readily employ other vectors not named but known to the art.

Viral vectors may be based on non-cytopathic eukaryotic viruses in which nonessential genes have been replaced with a target nucleotide sequence. Non-cytopathic viruses include retroviruses (*e*.*g*., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (*i.e.,* capable of directing synthesis of the desired proteins, but incapable of manufacturing an infectious particle). Such genetically altered retroviral expression vectors have general utility for the high-efficiency transduction of genes *in vivo.* Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are known in the art.
Other viral vectors include adeno-viruses and adeno-associated viruses, which are double-stranded DNA viruses that have also been approved for human use in gene therapy. The adeno-associated virus can be engineered to be replication deficient and is capable of infecting a wide range of cell types and species. Lentiviral vectors are a type of retrovirus that can infect both dividing and nondividing cells because their preintegration complex (virus "shell") can get through the intact membrane of the nucleus of the target cell. An exemplar lentiviral vector includes, but are not limited to a vector derived from HIV.

Other vectors include non-viral plasmid vectors, which have been extensively described in the art and are well known to those of skill in the art. See, *e.g.,* Sambrook et al. Molecular Cloning: A Laboratory Manual. Cold Spring Harbor Laboratory Press; 4th edition (June 15, 2012). Exemplary plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA.

In some embodiments, the stem cells involved in the methods and/or compositions described herein *(e.g.,* HSCs) have been genetically manipulated using a viral vector such as a retroviral vector or a lentiviral vector. Accordingly, in some embodiments, the stem cells involved in the methods and/or compositions described herein are gene-modified stem cells (*e.g.,* HSCs).

The genetic manipulation (*e*.*g*., transduction) of the stem cells is preferably performed when the stem cells are resting cells (non-cycling), i.e., cells that are not dividing. Such resting cells may in the quiescent G0 phase. Phenotypes of resting HSCs are known in the art and can be used to identify such HSCs for the methods and/or compositions described herein. For example, HSC-enriched population is at least CD34+CD38-CD90+. Like all somatic cells, stem cells (*e.g.*, HSCs) progress through the cell cycle, which is characterized by four phases: G1 (interphase), S (DNA synthesis phase), G2 (interphase) and M (mitosis phase). Stem cells (*e.g.*, HSCs) that proceed past the restriction point in the G1 phase enter the S phase, whereas those that do not pass the restriction point remain undivided. These undivided cells can withdraw from the cell cycle and enter the G0 phase: a state in which cells are termed quiescent or dormant. Such resting cells in the G0 phase can either reversibly re-enter the cell cycle and divide or remain dormant, losing the potential to cycle and, in some cases, becoming senescent. Quiescence is thus a property that characterizes stem cells (*e.g.,* HSCs) and allows them to maintain stemness of the cells. Without wishing to be bound by theory, when the stem cells (*e.g.,* HSCs) are genetically manipulated or modified before they enter a cell cycle (*e.g.,* when the cells are non-cycling) and are subsequently cultured in the presence of a p38 MAPK inhibitor, the p38 MAPK inhibitor delays the transition of the stem cells (*e.g.,* HSCs) to S phase, which may likely allow for stem cells (*e.g.,* HSCs) to repair any DNA damage, *e.g.,* induced by the genetic manipulation, thus retaining the stemness of the stem cells (*e.g.,* HSCs). This may be characterized by retained long term repopulating potential and/or a balanced lineage production. Accordingly, in some embodiments of the stem cells involved in the methods and/or compositions described herein, the stem cells (*e.g.,* HSCs) are non-cycling or non-dividing cells, *e.g.,* the stem cells are in the quiescent G0 phase.

Any of the stem cell populations described herein can be cultured in a suitable medium *(e.g.,* cell culture medium) in the presence of an effective amount of one or more p38 MAPK inhibitors as those described herein for a suitable period of time, *e.g.,* at least 18 hours, at least about 24 hour, at least 36 hours, at least 48 hours, at least 60 hours, at least 72 hours, at least 84 hours, at least 96 hours, at least about 5 days, at least about 6 days, at least about 7 days, or longer. In some embodiments, any of the stem cell populations described herein can be cultured in a suitable medium *(e.g.,* cell culture medium) in the presence of an effective amount of one or more p38 MAPK inhibitors as those described herein for about 18 hours to about 7 days, or about 1 day to about 7 days, or about 2 days to about 7 days, or about 3 days to about 7 days, or longer.

An "effective amount," "effective dose," or an "amount effective to", as used herein, refers to an amount of a p38 MAPK inhibitor as described herein that is effective in preserving at least one characteristic of the stemness (quiescence, repopulation potential, self-renewal potential, and multi-lineage differentiation potential) of stem cells, *e.g.,* HSCS, and/or results in a desired clinical effect, such as increased engraftment of HSCs in a subject after HSC transplantation. This can be monitored by routine methods or can be monitored according to the method for assessing engraftment of HSCs described herein. Effective amounts vary, as recognized by those skilled in the art, depending on, for example, the potency of the p38 MAPK inhibitor used.

For example, the effective amount of a p38 MAPK inhibitor for culturing the stem cells (*e.g.*, HSCs) in the methods described herein results in an increase in the proportion of stem cells (*e.g.*, HSCs) in the G0 quiescent phase by at least about 10% or more, including, *e.g.,* at least about 20%, at least about 30%, at least about 40%, at least about 50%, at least about 60%, at least about 70%, at least about 80%, at least about 90% or more, as compared to the proportion of stem cells (*e.g.*, HSCs) in the G0 quiescent phase without culturing in the presence of a p38 MAPK inhibitor. In some embodiments, the effective amount of a p38 MAPK inhibitor results in an increase in the proportion of stem cells (*e.g.*, HSCs) in the G0 quiescent phase by at least about 1.1-fold or more, including, *e.g.*, at least about 2-fold at least about 3-fold, at least about 4-fold, at least about 5-fold, at least about 6-fold, at least about 7-fold, at least about 8-fold, at least about 9-fold, at least about 10-fold or more, as compared to the proportion of stem cells (*e.g.*, HSCs) in the G0 quiescent phase without culturing in the presence of a p38 MAPK inhibitor.

In some embodiments, the effective amount of a p38 MAPK inhibitor used in the methods described herein results in a decrease in the proportion of the stem cells *(e.g.,* HSCs) in the S-G2-M phase before the first cell division cycle *(e.g.,* 24 hours) by at least about 10% or more, including, *e.g.,* at least about 20%, at least about 30%, at least about 40%, at least about 50%, at least about 60%, at least about 70%, at least about 80%, at least about 90% or more, as compared to the proportion of stem cells *(e.g.,* HSCs) in the S-G2-M phase before the first cell division cycle without culturing in the presence of a p38 MAPK inhibitor.

In some embodiments of the methods described herein, the stem cells *(e.g.,* HSCs) are cultured in the presence of a p38 MAPK inhibitor in an amount effective to reduce DNA damage or DNA double strand break (*e.g.*, as measured by the expression of γH2AX foci or 53bp1) in the stem cells (*e.g.*, HSCs) by at least about 20% or more, including, at least about 30%, at least about 40%, at least about 50%, at least about 60%, at least about 70%, at least about 80%, at least about 90% or more, as compared to the level of DNA damage or DNA double strand break measured in stem cells without culturing in the presence of a p38 MAPK inhibitor.

In some embodiments of the methods described herein, the stem cells *(e.g.,* HSCs) are cultured in the presence of a p38 MAPK inhibitor in an amount effective to reduce loss of long term repopulating potential (LTRP) *(e.g.,* as assessed in secondary transplant (2T)) by at least about 20% or more, including, at least about 30%, at least about 40%, at least about 50%, at least about 60%, at least about 70%, at least about 80%, at least about 90% or more, as compared to the LTRP when the cells are cultured without a p38 MAPK inhibitor. Examples 1-2 including Fig. 2 (panel B) describe an exemplary method to determine LTRP in 2T xenografts.

In some embodiments of the methods described herein, the stem cells *(e.g.,* HSCs) are cultured in the presence of a p38 MAPK inhibitor in an amount effective to reduce myeloid skewing bias in the stem cells by at least about 20% or more, including, at least about 30%, at least about 40%, at least about 50%, at least about 60%, at least about 70%, at least about 80%, at least about 90% or more, as compared to the myeloid skewing bias when the cells are cultured without a p38 MAPK inhibitor. Examples 1-2 including Fig. 2 (panel B) describe an exemplary method for multi-lineage reconstitution to assess myeloid skewing bias.

In some embodiments of the methods described herein, the effective amount of a p38 MAPK inhibitor for culturing the stem cells *(e.g.,* HSCs) results in a decrease in the phosphorylation level of at least one or more (including, *e.g.,* at least two, or at least three) members of p38 MAPK (*e.g.*, p38-α, p38-β, p38-γ, or p38-δ) in the stem cells (*e.g.*, HSCs), for example, by at least about 20%, including, for example, at least about 30%, at least about 40%, at least about 50%, at least about 60%, at least about 70%, at least about 80%, or more, as compared to the phosphorylation level of the corresponding member of p38 MAPK in the stem cells *(e.g.,* HSCs) without the treatment of the p38 MAPK inhibitor.

In some embodiments of the methods described herein, the effective amount of a p38 MAPK inhibitor does not substantially increase the phosphorylation level of ERK or JNK in the stem cells *(e.g.,* HSCs), for example, by no more than 20%, including, for example, no more than 10%, no more than 5%, no more than 3%, or lower, as compared to the phosphorylation level of ERK or JNK in the stem cells *(e.g.,* HSCs) without the treatment of the p38 MAPK inhibitor.

In some embodiments, the stem the stem cells *(e.g.,* HSCs), prior to transplantation *in vivo,* are cultured in a medium comprising an effective amount of a p38 inhibitor described herein, wherein the effective amount results in an increase in subsequent engraftment of stem cells *(e.g.,* HSCs) *in vivo* by at least about 10% or more, including, *e.g.,* at least about 20%, at least about 30%, at least about 40%, at least about 50%, at least about 60%, at least about 70%, at least about 80%, at least about 90% or more, as compared to engraftment of stem cells *(e.g.,* HSCs) without culturing the cells with a p38 MAPK inhibitor prior to transplantation. In some embodiments, the effective amount of a p38 MAPK inhibitor results in an increase in subsequent engraftment of stem cells *(e.g.,* HSCs) by at least about 1.1-fold or more, including, *e.g.,* at least about 2-fold at least about 3-fold, at least about 4-fold, at least about 5-fold, at least about 6-fold, at least about 7-fold, at least about 8-fold, at least about 9-fold, at least about 10-fold or more, as compared to engraftment of stem cells *(e.g.,* HSCs) without culturing the cells with a p38 MAPK inhibitor prior to transplantation.

An effective dose of a p38 MAPK inhibitor for the methods described herein can be at least about 10 nM, at least about 20 nM, at least about 30 nM, at least about 40 nM, at least about 50 nM, at least about 100 nM, at least about 200 nM, at least about 300 nM, at least about 400 nM, at least about 500 nM, at least about 600 nM, at least about 700 nM, at least about 800 nM, at least about 900 nM, at least about 1 µM, at least about 2 µM, at least 3 µM, at least about 4 µM, at least about 5 µM, at least about 6 µM, at least about 7 µM, at least about 8 µM, at least about 9 µM, or at least about 10 µM. In some embodiments, the effective dose of a p38 MAPK inhibitor for the methods described herein can be no more than 10 µM, no more than 9 µM, no more than 8 µM, no more than 7 µM, no more than 6 µM, no more than 5 µM, no more than 4 µM, no more than 3 µM, no more than 2 µM, no more than 1 µM, no more than 900 nM, no more than 800 nM, no more than 700 nM, no more than 600 nM, no more than 500 nM, no more than 400 nM, no more than 300 nM, no more than 200 nM, no more than 100 nM, no more than 50 nM, no more than 40 nM, no more than 30 nM, no more than 20 nM, or no more than 10 nM. Combinations of the above-referenced ranges are also possible. For example, an effective dose of a p38 MAPK inhibitor for the methods described herein can be about 30 nM to about 10 µM, or about 100 nM to about 5 µM, or about 400 nM to about 800 nM.

The stem cells can preserve their stemness when they are cultured in the presence of a p38 MAPK inhibitor according to the methods described herein. In some embodiments, the percentage of pluripotent stem cells after the *ex vivo* culturing process is at least 70% *(e.g.,* 80%, 90%, 95%, 97%, or above) of that before the *ex vivo* culture. In other embodiments, less than 30% (*e.g.*, less than 25%, 20%, 15%, 10%, or 5% or less) of the stem cells would differentiate, *e*.*g*., from pluripotent stem cells to multipotent stem cells, or from multipotent cells to specialized cells, during the *ex vivo* culture process described herein. The presence of different types of stem cells, *e.g.,* pluripotent stem cells and multipotent cells, and specialized cells in the *ex vivo* culture can be monitored via a routine method, for example monitored by the presence of cell surface markers specific to a specific type of stem cells or specific to a specialized cell.

In some embodiments, adult HSCs are subjected to the *ex vivo* culturing process described herein, which involves the use of one or more p38 MAPK inhibitors. The percentage of HSCs after the culturing may be at least 70% *(e.g.,* 80%, 90%, 95%, or higher) of that of HSCs prior to the culturing. Alternatively or in addition, the percentage of hematopoietic progenitor cells (HPCs) in the cells after the *ex vivo* culture may be lower than 30% (*e.g.*, lower than 25%, 20%, 15%, 10%, or 5%).

### III. Stem Cell Therapy

The stem cells prepared by the *ex vivo* culturing methods described herein can be used in stem-cell therapy, which is the use of stem cells to treat or prevent a disease or condition, including, for example, neurodegenerative diseases and conditions, diabetes, heart disease, and other conditions. Examples of suitable conditions to be treated by stem cell therapy include, but are not limited to, acute myeloid leukemia (AML), chronic myeloid leukemia (CML), acute lymphoblastic leukemia (ALL), Hodgkin lymphoma, Non-Hodgkin lymphoma, neuroblastoma, Ewing sarcoma, Myelodysplastic syndromes, Gliomas, and other solid tumors. Stem cell therapy can also be applied to non-malignant conditions such as thalassemia, aplastic anemia, Fanconi anemia, immune deficiency syndromes, or inborn errors of metabolism. In some embodiments, the HSCs prepared by the *ex vivo* culturing methods described herein can be used for transplantation in treatment of hematopoietic disorders, including, but not limited to, acute myeloid leukemia (AML), chronic myeloid leukemia (CML), acute lymphoblastic leukemia (ALL), chronic lymphocytic leukemia (CLL), juvenile myelomonocytic leukemia, Hodgkin lymphoma, and Non-Hodgkin lymphoma.

Hematopoietic stem cell transplantation (HSCT) is the transplantation of multipotent hematopoietic stem cells, usually derived from bone marrow, peripheral blood, or umbilical cord blood. In some instances, the HSCs can be autologous (the patient's own stem cells are cultured by the *ex vivo* culturing methods described herein and used for treating a disease). In other examples, the HSCs cay be allogeneic (the stem cells come from a donor and is then cultured by the *ex vivo* culturing methods described herein). Such HSCs can be used for treating certain cancers of the blood or bone marrow, such as multiple myeloma or leukemia. In these cases, the recipient's immune system is usually destroyed with radiation or chemotherapy before the transplantation.

In some examples, the HSCs described herein (*e*.*g*., human adult HSCs) can be genetically engineered to express a γ globin for use in treating anemia, such as sickle cell anemia and thalassemia. See, *e.g.*, US20110294114 and WO2015/117027.

In any of the stem cell therapy described herein, suitable stem cells can be collected from the *ex vivo* culturing method described herein and mixed with a pharmaceutically acceptable carrier to form a pharmaceutical composition, which is also within the scope of the present disclosure.

To perform the treatment methods described herein, an effective amount of the stem cells can be administered into a subject in need of the treatment. The stem cells may be autologous to the subject. Administration of autologous cells to a subject may result in reduced rejection of the stem cells as compared to administration of non-autologous cells. Alternatively, the stem cells are allogeneic cells. For example, allogeneic stem cells may be derived from a human donor and administered to a human recipient who is different from the donor.

In some embodiments, the stem cells can be co-used with a therapeutic agent for a target disease, such as those described herein. The efficacy of the stem cell therapy described herein may be assessed by any method known in the art and would be evident to a skilled medical professional. Determination of whether an amount of the cells or compositions described herein achieved the therapeutic effect would be evident to one of skill in the art. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. In some embodiments, the effective amount alleviates, relieves, ameliorates, improves, reduces the symptoms, or delays the progression of any disease or disorder in the subject.

### General Techniques

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook, et al., 1989) Cold Spring Harbor Press; Oligonucleotide Synthesis (M. J. Gait, ed., 1984); Methods in Molecular Biology, Humana Press; Cell Biology: A Laboratory Notebook (J. E. Cellis, ed., 1998) Academic Press; Animal Cell Culture (R. I. Freshney, ed., 1987); Introduction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds., 1993-8) J. Wiley and Sons; Methods in Enzymology (Academic Press, Inc.); Handbook of Experimental Immunology (D. M. Weir and C. C. Blackwell, eds.); Gene Transfer Vectors for Mammalian Cells (J. M. Miller and M. P. Calos, eds., 1987); Current Protocols in Molecular Biology (F. M. Ausubel, et al., eds., 1987); PCR: The Polymerase Chain Reaction, (Mullis, et al., eds., 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: a practical approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J. D. Capra, eds., Harwood Academic Publishers, 1995).

### EXAMPLES

### Example 1: Development of an Animal Model to Study Adult Human HSC and the Effect of Ex Vivo Manipulation

The goals of the present study were to develop a robust animal model of human HSC in a xenograft model that mimics human clinical trial data, to study the mechanism of HSC loss during *ex vivo* culture and gene transfer, and to use small molecule inhibitors of identified pathways to reverse the HSC loss during *ex vivo* culture and/or genetic manipulation.

*Ex vivo* culture of HSC may activate stress signaling (p38 MAPK) in HSC, resulting in either asymmetric HSC division or symmetric HPC commitment at the expense of HSC self-renewal. Vector integration events further instigates a DNA damage response that results in their differentiation to HPC. Thus, using a p38 MAPK inhibitor may reverse HSC loss during *ex vivo* culture and/or genetic manipulation *(e.g.,* vector integration).

### Development of Adult Human Mesenchymal Stem Cell (MSC) Model

### (i) Myelo-ablative Conditioning

Two recent gene therapy trials exploring myelo-ablative conditioning have been performed (Table 3). Kang et al., Blood (2010); Cavazzana-Calvo et al., Nature (2010) About 4-70 million CD34⁺ cells were infused in the studies, and among them, the number of transduced CD34⁺ cells infused ranged from 1-30 million. When the numbers were extrapolated in terms of transduced HSCs, about 13,000 to 300,000 were infused, but this resulted in a long term engraftment of about 0-1%, showing tremendous loss.

**Table 3: Summary of Recent Myelo-ablative Conditioning Studies**

| **Gene Transfer Trial** | **Type of Vector** | **CD34+ Cells Infused/kg** | **tCD34+ Infused/kg** | **[tHSC infused/kg ]** | **Short Term Engraftmen t** | **Long Term Engraftmen t** |
|---|---|---|---|---|---|---|
| **CGD** | RV | 43 × 10⁶ | 31 × 10⁶ | (3 × 10⁵) | 26% | 1% |
| | | 71 × 10⁶ | 29 × 10⁶ | (3 × 10⁵) | 5% | 0% |
| | | 19 × 10⁶ | 5 × 10⁶ | (5 × 10⁴) | 4% | 0.03% |
| **β-thalassemia** | LV | 4 × 10⁶ | 1.3 × 10⁶ | (1.3 × 10⁴) | 1.3% | 0.0018% |

### (ii) Xenograft Model of Adult Human HSCs

A xenograft model was developed to study hHSC engraftment. In the xenograft model of adult human HSC, CD34⁺ cells were derived and isolated from fresh MPB. The fresh CD34⁺ cells were either directly transplanted into immunocompromised NSG mice or cultured with human cytokines and then transduced either with lentivirus or retrovirus. For the lentivirus (LV) vector, the cultured cells were transduced for 18-24 hours and harvested and injected into NSG mice after 18-24, 36-42, and 72-96 hours of *ex vivo* culture. For the retrovirus (RV) vector, the cells were injected into NSG mice after 72-96 hours of *ex vivo* culture. The multipotential human hematopoietic progeny (hCD45⁺) following huCD34⁺ transplant in NSG mice were then measured to confirm that the model human HSC engraftment from adult (mobilized peripheral blood) CD34⁺ HSC/P that mimics the results observed in gene therapy clinical trials. Human cell engraftment in the bone marrow of NSG mice at 6, 12, and 24 weeks post-primary transplant (1T), and 6 weeks post-secondary transplant (2T) of fresh/cultured human CD34⁺ cells. Cells were transduced with lentiviral (LV) or retroviral (RV) vector.

It was observed that increased time in culture leads to HSC division, and that a portion of the transduced HSCs undergo apoptosis. The increased time of *ex vivo* manipulation of human HSC does not affect engraftment in primary NSG mice, even at 6 months, but does lower secondary engraftment. Thus, increased time of ex vivo manipulation of human HSCs (hHSC) results in loss of long-term engraftment potential of hHSC in a xenograft model. The loss of secondary engraftment was found to correlate with HSPC and HSC division, and a portion of HSC (CD34+38-90+) with vector integration were observed *via* flow cytometry to undergo apoptosis. The apoptotic population has high GFP expression in flow cytometry studies, suggesting the high vector copies or increased double strand breaks (DSBs) that occur from vector integration are not well-tolerated by HSCs.

### Mechanisms of HSC loss during ex vivo culture and gene transfer

The loss of secondary engraftment was found to correlate with HSPC and HSC division, as extended cultures result in both HSPC and HSC division. In addition, p38 MAPK activation increases as HSCs progress through the cell cycle.

Next, the *ex vivo* cultured cells were subjected to flow cytometry for further characterization. It was observed that an increased time in culture showed an increase in p-p38 MAPK level in human HSCs. Human HSCs can be characterized as CD34+CD38lowCD90+CD45RA-CD49f+ There also appeared to be slight increase in ERK at 72 hours.

Flow cytometry also demonstrated that p38MAPK inhibitors can prevent HSC loss and may even retain HSC status through cell division during *ex vivo* cultures. Four different p38 inhibitors were shown to increase the percent of hHSCs *ex vivo* (Fig. 3). One p38 inhibitor, BIRB-796, was selected for further analysis. It was observed that BIRB-796 decreased p-p38 in hHSCs in *ex vivo* cultures, resulting in a significant increase in hHSCs in culture (Fig. 4).

p38 inhibition was also shown to significantly increase the number of 2T mice (6 weeks post-secondary transplant) with any human cell engraftment while not affecting the overall human cell engraftment in the 1T mice (except for lentivirus-transduced cells cultured for 72 hours). For engraftment in 1T model, hCD45+ cell engraftment in bone marrow of 1T mice at 24 weeks following transplant of culture hCD34+ cells with or without p38 inhibitor BIRB-796 were assessed.

p38 inhibition, e.g., with BIRB-796, was also shown to maintain the G0 quiescent phase of both untransduced and transduced HSCs during the early time period of *ex vivo* culture. Ki67, a nuclear antigen associated with cell proliferation, was used to determine cell cycle status via flow cytometry; negative stains were considered to represent quiescent HSCs.

Data from 10 experiments have demonstrated that lentivirus and retrovirus transduce HSC and HPC comparably.

Primary transplant (1T) mice (n=8-16) were analyzed 24 weeks after their respective transplants with respect to HPC progeny. A multilineage analysis of the harvested cells demonstrated that genetic manipulation of CD34⁺ cells during *ex vivo* culture shows a loss of lymphoid potential and myeloid skewing of the long term *in vivo* progeny. p38MAPK inhibition resulted in long term repopulating ability, but does not revert the effect.

p38 inhibition maintains the quiescent phase of HSCs and reduces DNA damage response (DDR) early in culture. Cell cycle entry and gene manipulation induces a DDR in human HSCs that is reduced by inhibiting p38MAPK early in culture. DNA double-strand breaks in Human HSCs were assessed by γ-H2AX staining, a sensitive and specific indicator of DNA double-strand breaks.

### Summary

HSC division in culture decreases the self-renewing potential of adult human HSC read out in secondary transplant. Some HSCs with a high level transduction undergo apoptosis.

Increased time in culture has been demonstrated to activate p38MAPK in human HSC, while the inhibition of p38MAPK during *ex vivo* cultures appears to maintain or increase the phenotypic HSC *in vitro.* Functionally, inhibition of p38MAPK during *ex vivo* manipulation and gene transfer retains human HSC engraftment in secondary transplant mice. p38 inhibition may exert its effect by maintaining HSC quiescence during *ex vivo* culture manipulation.

### Example 2: Mechanisms of Human Hematopoietic Stem Cell Loss during Ex Vivo Manipulation and Gene Transfer

Hematopoietic stem cells (HSCs) are ideal targets for gene therapy for several inherited hematological diseases including hemoglobinopathies. Gene transfer into HSC involves in a 2-4 day *ex-vivo* culture, making these cells less competent at engraftment *in vivo.* This necessitates large transduced HSC (tHSC) doses and myelo-ablative conditioning to destroy resident HSCs. In this Example, the transduced human CD34⁺ HSPC were modeled with long term repopulating potential (LTRP) in NOD.LtSz-scid Il2rγ-/- (NSG) mice using primary and secondary transplants, with B, Myeloid and T cell reconstitution. Mobilized peripheral blood CD34⁺ cells (containing adult hematopoietic stem cells) were used, since these are the relevant targets for gene therapy and their engraftment kinetics vastly differ from cord blood CD34⁺ cells, which are commonly used in the NSG mouse model. Gene transfer into hHSPCs by lentivirus (LV) or γ-retrovirus vectors (RV) followed by engraftment into NSG mice revealed results similar to those seen in gene therapy trials, showing that extended time of *ex-vivo* manipulation of hCD34⁺ cells results in huge loss of the LTRP in secondary transplanted mice. Furthermore, genetically manipulated CD34⁺ cell progeny transduced in 3-4 day culture conditions showed relative loss of lymphoid potential at the expense of increased myeloid lineage skewing, which is specific to transduced cells. Mechanistically, extended *ex vivo* culture and gene manipulation induced a DNA damage response (DDR) in hHSCs as revealed by increased γ-H2AX and 53BP1 signaling that is associated with activation of p38^{MAPK}; this effect was reversed by use of a p38 inhibitor, which helped retain the LTRP and engraftment in secondary transplanted NSG mice and partially reverted the myeloid skewing phenotype.

### Prolonged ex vivo culture of human HSPCs results in substantial loss of LTRP and a myeloid skewed gene-modified progeny

Gene transfer into HSCs requires cytokine rich culture conditions for therapeutically relevant gene transfer. Girard-Gagnepain, A., et al., Blood (2014) 124(8): p. 1221-31. In order to investigate the effect of *ex vivo* culture and genetic manipulation on the long term repopulating potential (LTRP) of adult human CD34⁺ hematopoietic stem/progenitor cells (HSPC), the NOD.Cg-*Prkdc*^{scid}Il2rg^{tm1wjl}/SzJ mouse model (Jackson Laboratory; Mouse Strain Datasheet ― 005557), also known as NOD/SCID/IL2rg null or NSG xenograft model, was adapted to an adult HSC model, since the majority of the studies in this model are based on umbilical cord blood-derived (CB) CD34+ cells; and adult CD34+ cells do not engraft sufficiently, or sustain a secondary graft at CD34+ cell doses used for CB CD34+ cells in this model (data not shown). One million CD34+ cells from G-CSF mobilized peripheral blood (MPB) were transplanted in irradiated NSG mice, and serial engraftment in the bone marrow (BM) of the animals at 6, 12 and 24-weeks was assessed [circulating human cells do not correlate with engraftment in BM (Fig. 6, panels A-B)]. A multipotential graft composed of B-, T- and myeloid cells was only evident by 24-weeks in primary mice transplanted with MPB CD34+ cells (Fig. 6, panels C and D); a 1:1 secondary transplant (2T) performed at 6, 12 and 24-weeks from primary transplant (1T) mice only yielded successful human secondary engraftment (>0.01% human CD45+ cells) from the 24 week 1T mice. Hence, this model of adult human hematopoiesis can assess multipotential hematopoiesis at 6 months in 1T xenografts and LTRP in 2T xenografts.

Two protocols for lentivirus vector (LV) and γ-retrovirus vector (RV) gene transfer, commonly used for clinical transductions were used: MPB CD34+ HSPCs were transduced with (a) GFP-encoding LV for 18-24 hours (18-24h) and either immediately transplanted, or kept in culture for an additional 12-16h and transplanted at 36-42h into irradiated NSG mice; (b) alternatively, CD34+ cells were pre-stimulated for 2 days *ex vivo,* and then transduced with a self-inactivating GFP-encoding RV at day 2 and 3, and transplanted into NSG mice between 72-96h. Notably, gene editing also requires similar conditions, the former for gene disruption or knockout (24h or 36h) using the cellular NHEJ repair pathway, and the latter conditions for homology directed repair, that requires cycling HSC. As a control, NSG mice were transplanted with unmanipulated CD34+ HSPCs immediately following their isolation (0h). Variations in *ex vivo* culture time or time of exposure to vectors were made in some experiments, to obtain appropriate controls (Fig. 7). At 24 weeks after 1T, robust human hematopoietic engraftment (human CD45+ cells) was observed in the BM under all ex-vivo culture and gene transfer conditions, with no major variations (Fig. 8, panel A). However, there was a significant loss of LTRP in 2T animals when cells were cultured for greater than 24h, the loss being greatest in the RV 72-96h group (Fig. 8, panel B). Gene transfer efficiency in CD34+ cells *in vitro* (using the conventional colony forming assay) showed that both RV and LV transduced CD34+ cells comparably in this assay (Fig. 7). However, while there was a slight decrease in LV-transduced (GFP+) human cells (from an average of 80% *in vitro,* to 50-60% by 24-weeks *in vivo),* there was a significant and progressive reduction in RV-transduced (GFP+) cells to 5-8% *in vivo* (Fig. 7). Hence, RV-transduced human graft was lost with time in the NSG mice, while that transduced with LV vectors was sustained in the animals at 6 months.

Next, the multi-lineage reconstitution was analyzed at 6, 12 and 24 weeks in BM of 1T animals, both of the untransduced (GFP⁻) versus transduced (GFP⁺) progeny. Similar lineage proportions were observed (mostly B-myeloid in both the GFP+ and GFP-populations), comparable to the lineage pattern of unmanipulated CD34+ cell (0h) derived graft at 6-weeks post 1T. However, by 12-weeks, the 72-96h RV-gene modified population showed a significant increase in myeloid cells, which occurred at the expense of reduced B cell progeny (Fig. 9; Figs. 20- 21); and by 24 weeks, the myeloid bias was also apparent in the 36-42h LV-transduced human grafts, and this bias was extreme in the 72-96h RV-transduced grafts, at the expense of significantly reduced B and T lymphoid population; there were also significantly less GFP+ CD34+ cells in both the 36-42h LV and 72-96h RV grafts (Fig. 8, panels C-J). In addition, there was no lineage skewing in the untransduced cells in any of the tested conditions, indicating that this was not an effect of the cytokine/culture conditions, where the untransduced lineage pattern was similar to the human graft derived from unmanipulated CD34+ cells (0h). These data demonstrate that myeloid lineage bias was secondary to transduction and increased time in culture. It is noted that the RV used in this Example had a self-inactivating design and driven by a cellular promoter EF1α, so that it is devoid of its long terminal repeat (LTR) enhancers, known to cause insertional genotoxicity and potentially skew lineages by influencing expression of genes flanking the insertion site, and has not shown the insertional adverse events observed with conventional (LTR-intact) RV in experimental models (Thornhill, S.I., et al., Mol Ther (2008) 16(3): p. 590-8; Zychlinski, D., et al., Mol Ther (2008) 16(4): p. 718-25) and in human trials (Hacein-Bey-Abina, S., et al., N Engl J Med (2014) 371(15): p. 1407-17; De Ravin, S.S., et al., Sci Transl Med (2016) 8(335): p. 335ra57). The LV vector used has also been tested in human trials with no adverse events from vector insertion. Cartier, N., et al., Science (2009) 326(5954): p. 818-23. Collectively, *ex-vivo* culture beyond the first 24h and gene transfer results in loss of ability to repopulate hematopoiesis in 2T (LTRP) and the remaining transduced progeny is myeloid-biased, at the expense of the lymphoid progeny. The model also reflects the results of numerous RV gene therapy trials, where there was modest success despite robust gene transfer *in vitro* (Kang, E.M., et al., Blood (2010) 115(4): p. 783-91) or only short term engraftment. In addition, the model also simulates some successful LV gene therapy trials that have shown stable gene marking with short-term gene transfer (Cartier, N., et al., Science (2009) 326(5954): p. 818-23; Biffi, A., et al., Science (2013) 341(6148): p. 1233158; Aiuti, A., et al., Science (2013) 341(6148): p. 1233151; and Cavazzana-Calvo, M., et al., Nature (2010) 467(7313): p. 318-22), but modest success to failure in other LV trials where gene transfer was performed at 72-96h, validating this model as a preclinical model for adult human HSC gene transfer.
Mechanisms for the loss of LTRP and myeloid-lineage biased progeny were investigated. RV only transduce dividing cells while LV can transduce both dividing and non-dividing cells, although LV also preferably transduce dividing cells. Lewis, P.F. and M. Emerman, J Virol (1994) 68(1): p. 510-6. Hence, it is assumed that the failure of RV trials was from preferential integration of transgenes into hematopoietic progenitor cells (HPCs) or multipotent progenitors (MPP) that comprise 99% of the CD34+ HSPC population, and poor transduction of the quiescent HSC population. Using the phenotype of human HSC identified by Dick and coworkers (Notta, F., et al., Science (2011) 333(6039): p. 218-21), the clinical transduction conditions were found to be optimized to transduce human HSC (CD34+CD38-CD90+CD45RA-CD49f+), (MPP, defined as CD34+CD38-CD90-CD45RA- *(Id.;* Huntsman, H.D., et al., Blood (2015) 126(13): p. 1631-3)) and total CD34+ cells comparably with RV and LV (Fig. 10), but transduced HSC either die or change fate to HPC with gene transfer. A careful assessment of the HSPC and HSC compartments showed no loss of viability of CD34⁺ cells or apoptosis in the HSC subpopulation within them *ex vivo;* in fact there was an increase in percent phenotypic HSCs with increasing time in culture (Fig. 11, panels A-C). It was also observed that the HSC-enriched population began entering S-phase only after 24h of *ex vivo* culture, with a large proportion were in cycle by 72h. It was hypothesized that HSCs are physiologically maintained in their hypoxic niches (Kubota, Y., et al., Biochem Biophys Res Commun (2008) 366(2): p. 335-9), inducing cell division in culture in normoxic conditions likely induces high oxidative stress. Mantel, C.R., et al., Cell (2015) 161(7): p. 1553-65. Indeed, reactive oxygen species (ROS) were significantly increased in CD34+CD38-CD90+ (HSC-enriched) population with 72-96h of culture, and it appeared that ROS were generated from mitochondria (Fig. 11, panels D-F). N-acetylcysteine amide was able to decrease ROS in the HSC-enriched population, but resulted in a reciprocal decline in gene transfer efficiency (Fig. 11, panels G-H). Therefore, the downstream pathways activated by increased oxidative stress were investigated. ROS has been shown to induce stress signaling pathways, especially mitogen-activated protein kinases (MAPKs). Ito, K., et al., Nat Med (2006) 12(4): p. 446-51. The phosphorylation status of ERK, JNK and p38 MAPKs was analyzed in the different conditions and a significant activation of only of p38 MAPK (p38) with *ex vivo* culture beyond 24h was found (Fig. 12, panels A-F; Fig. 22). Interestingly, there was increased activation p38 in cycling HSC; although transduced HSC did not have higher p38 activation (Fig. 12, panels G-H; Fig. 22). A previous study has shown that CB CD34⁺ cells cultured for a week resulted in increased p38 activation. Zou, J., et al., Ann Hematol (2012) 91(6): p. 813-23. Hence, the loss of human HSC LTRP may be due to extended time in culture rather than LV or RV transduction. To ensure that this is the effect specific to just p38 MAPK, various MAPK inhibitors (McGuire, V.A., et al., Mol Cell Biol (2013) 33(21): p. 4152-65; Campbell, R.M., et al., Mol Cancer Ther (2014) 13(2): p. 364-74) were used in CD34+ cell cultures for 72h and observed decreased p38 activation in HSC enriched population (Fig. 12, panels J-K; Fig. 22).

Among these p38 inhibitors (p38i), Birb-796, a highly selective p38α inhibitor (Pargellis, C., et al., Nat Struct Biol (2002) 9(4): p. 268-72), was used for further studies. p38i was able to significantly lower p-p38 levels in the HSC population to that seen in unmanipulated HSC at all the different time points in culture and transductions (Fig. 13, panels A-B). Serial transplants of human CD34⁺ HSPCs were then performed with or without p38i, to assess its effect on LTRP and on myeloid lineage bias. The human CD34⁺ HSPCs were transduced with LV for the first 18-24h, and kept in culture for 24, 46-42 or 72-96h before the transplant, or transduced with RV at 44h and 68h and transplanted at 72-96h (Fig. 7). At 24 weeks after 1T, human CD45⁺ cell engraftment in the NSG BM was similar among all the groups without p38i (Fig. 13, panel C) and slightly improved with p38i for the 72-96h LV and RV groups.

Remarkably, however, p38i at all time points was able to retain the secondary engraftment potential or long term repopulating potential (LTRP) to the levels observed after transplanting unmanipulated CD34+ cells (Fig. 13, panel D, and Fig. 14): nearly a third of the mice do not have LTRP when CD34+ cells were in culture for longer than 24h without p38i, including HSPC transduced with LV within 24h but kept in culture for 72-96h or those transduced with RV after 2 days in culture. But addition of a p38i during the culture period restores the LTRP in 2T.

With p38i exposure during *ex vivo* culture, the myeloid lineage skewing was restored to levels similar to untransduced cells or 0h controls if HSPC were transduced within the first 24h with LV, even if they were kept in prolonged cultures for up to 72-96h. However, in the 72-96h RV group, where myeloid skewing was most pronounced at the expense of the lymphoid lineage and CD34+ cells without p38i, there was some improvement in the skewing, but it was still not lineage balanced. Significant increase in myeloid progeny, with a reduction in B lymphoid cells and CD34+ cells was still observed, and there was significant loss of human HSPCs (Fig. 13, panels I-J), increased myeloid skewing (Fig. 13, panels E-F) at the cost of B-lymphoid cells (Fig. 13, panels G-H) in the RV transduced population.

Hence, lineage skewing were specific to cells that were transduced when they were dividing. p38i was able to significantly revert this phenotype but only partially (Fig. 13, panels E-J). Furthermore, p38i had no effect in gene (GFP) transfer efficiency, *in vitro and in vivo* at 6 and 24 weeks post 1T (Fig. 15, panels A-C). Taken together, the results indicate that the inhibition of p38 can maintain HSCs during *ex vivo* culture conditions for gene transfer which were otherwise lost due to the activation of *ex vivo* cytokine rich culture and transduction induced stress.

### P38 inhibition retained HSCs in quiescent phase during early ex vivo culture and reduceed DNA damage response

The mechanism by which p38 inhibition is able to retain the LTRP of cultured and transduced HSCs was next investigated. There was no significant change in the total number of CD34+ cells or their viability with or without p38i. The HSC enriched CD34⁺ 38⁻ 90⁺ population showed no difference in apoptosis, ROS levels or transduction efficiency (Fig. 16, panels A-D), and inhibition of p38 increased the percentage of phenotypic HSC (Fig. 16, panel E).

Since the transition of quiescent HSCs into cell cycle has been shown to trigger the DNA damage response (DDR) and repair pathway (Walter, D., et al., Nature (2015) 520(7548): p. 549-52), whether p38 activation that occurs with HSC division mediates the DDR pathway in *ex vivo* cultured and transduced HSPCs was examined. Indeed, increased γH2AX foci were found in the CD34⁺ CD38⁻ CD90⁺ HSC enriched population that were cultured and transduced for either 36-42 hours or 72 hours; such DNA damage response was rescued by p38 inhibition (Fig. 17, panels A-B). In addition, 53bp1 staining was concurrently performed to ensure that increased γH2AX associated with DDR foci (Fig. 17, panel C). The DDR was confirmed by flow cytometry, where a progressive increase in γH2AX levels with increased time in culture was observed, and activation of DDR in transduced versus untransduced cells was examined. Vector integration occurs after a DNA double strand break, and hence can evoke a DDR. Skalka, et al., Cell Death Differ (2005) 12 Suppl 1: p. 971-8. As compared to unmanipulated HSC (0h), increased γH2AX staining was seen at 24 hours, which returned to levels seen in unmanipulated HSC with p38i treatment (Fig. 17, panels D-F). The GFP protein expression is not present at the 24h time point, since it is soon after transduction. Notably after 36 hours, the untransduced (GFP-) versus transduced (GFP+) population could be separately analyzed. The transduced (GFP+) population had higher γH2AX than the untransduced (GFP-) population both in the 36-42h and 72-96h group; and p38i significantly reduced the percentage of yH2AX+ cells, although levels did not return to those seen in 0h HSC, both the percentage of HSC that stain for γH2AX (Fig. 17, panel F) as compared to 0h HSC (Fig. 17, panel D), and the γH2AX MFI (Fig. 17, panels D-E). This indicates that p38 inhibition significantly reduces the DDR that occurs in HSC both with increasing time in culture and with transduction, although it does not reduce it to baseline levels in 0h HSC after 24h of culture and transduction.

The role of p38i on HSC cell cycle was next examined, since transition of quiescent HSCs into cell cycle has been shown to trigger the DDR pathway. Walter, D., et al., Nature (2015) 520(7548): p. 549-52. Nearly all HSC were in G0 phase when freshly isolated from MPB. By 24h, nearly a third of them transitioned to the G1 phase. p38i significantly increased the proportion of HSC in the G0 quiescent phase, and decreased the proportion of HSC in the S-G2-M phase before the first HSC division (24h). However, after 24h, as HSC progressed through cell cycle, the effect of p38i was lost. Hence, the data suggests that when the transduction occurs at the first 24 hours (when HSC are non-cycling), p38i-mediated delay in transition to S phase (Fig. 17, panels G-H and Fig. 18) likely allows for HSCs to repair the DNA damage, and repair of the DDR results in retention of HSC fate, as shown by retained LTRP and a balanced lineage production. However, when HSC are transduced when actively cycling (SG2-M phase), as with RV, the effect of p38i is lost (Fig. 17, panels G-H and Fig. 18), and there is significant loss of LTRP of the transduced HSC; moreover, transduction of cycling HSC causes them to produce a myeloid-biased progeny, reminiscent of aged HSC. This phenomenon is not restricted to MPB-derived HSC, but also seen in HSC derived from adult human bone marrow (Fig. 19).

In sum, these results can be utilized to develop a clinical gene therapy protocol to prevent the loss of gene-modified hHSCs and improve the overall engraftment.

## Claims

1. A method for preserving engraftment activity of hematopoietic stem cells (HSCs) undergoing an ex vivo genetic manipulation, comprising:
i) performing the ex vivo genetic manipulation of the HSCs in quiescent G0 phase; and
ii) culturing the genetically modified HSCs produced in step (i) in a medium containing a p38 mitogen-activated protein kinase (MAPK) inhibitor.

2. The method of claim 1, wherein the genetic manipulation comprises transduction of an integrating vector or wherein the genetic manipulation comprises genome editing.

3. The method of any one of claims 1 or 2, wherein the HSCs are obtained from a subject, optionally wherein the subject is a human subject.

4. The method of claim 3, wherein the HSCs are adult HSCs obtained from bone marrow or peripheral blood cells of the human subject, or wherein the HSCs are obtained from umbilical cord blood cells of the human subject.

5. The method of claim 2, wherein the integrating vector is a viral vector, optionally wherein the viral vector is a retroviral vector or a lentiviral vector.

6. The method of claim 1 or 2, wherein the HSCs are human adult HSCs.

7. The method of any one of claim 6, wherein the human adult HSCs are obtained from the bone marrow or peripheral blood cells.

8. The method of any one of claims 1-7, wherein the MAPK inhibitor is doramapimod, ralimetinib, an aminopyridine-based, ATP-competitive inhibitor of p38 MAPK, or a pyridinyl imidazole inhibitor.

9. The method of any one of claims 1 to 8, wherein the culturing is performed for 1 to 7 days.

## Patentansprüche

1. Verfahren zum Bewahren einer Engraftmentaktivität von blutbildenden Stammzellen (hematopoietic stem cells ― HSCs), die einer ex vivo genetischen Manipulation unterzogen werden, das Folgendes umfasst:
i) Durchführen der ex vivo genetischen Manipulation der HSCs in einer ruhenden G0-Phase; und
ii) Kultivieren der genetisch modifizierten HSCs, die in Schritt (i) hergestellt werden, in einem Medium, das einen p38-Mitogen-aktivierten-Proteinkinase(MAPK)-Inhibitor enthält.

2. Verfahren nach Anspruch 1, wobei die genetische Manipulation eine Transduktion eines integrierenden Vektors umfasst oder wobei die genetische Manipulation ein Genom-Editieren umfasst.

3. Verfahren nach einem der Ansprüche 1 oder 2, wobei die HSCs von einem Subjekt erhalten werden, optional wobei das Subjekt ein menschliches Subjekt ist.

4. Verfahren nach Anspruch 3, wobei die HSCs erwachsene HSCs sind, die aus Knochenmark oder peripheren Blutzellen des menschlichen Subjekts erhalten werden, oder wobei die HSCs aus Nabelschnurblutzellen des menschlichen Subjekts erhalten werden.

5. Verfahren nach Anspruch 2, wobei der integrierende Vektor ein viraler Vektor ist, optional wobei der virale Vektor ein retroviraler Vektor oder ein lentiviraler Vektor ist.

6. Verfahren nach Anspruch 1 oder 2, wobei die HSCs menschliche erwachsene HSCs sind.

7. Verfahren nach einem der Ansprüche 6, wobei die menschlichen erwachsenen HSCs aus dem Knochenmark oder den peripheren Blutzellen erhalten werden.

8. Verfahren nach einem der Ansprüche 1-7, wobei der MAPK-Inhibitor Doramapimod, Ralimetinib, ein ATP-kompetitiver Inhibitor auf Aminopyridin-Basis von p38-MAPK oder ein Pyridinylimidazol-Inhibitor ist.

9. Verfahren nach einem der Ansprüche 1 bis 8, wobei das Kultivieren 1 bis 7 Tage lang durchgeführt wird.

## Revendications

1. Procédé permettant de conserver l'activité de prise de greffe de cellules souches hématopoïétiques (CSH) subissant une manipulation génétique ex vivo, comprenant :
i) la réalisation de la manipulation génétique ex vivo des CSH en phase G0, phase de quiescence ; et
ii) la culture des CSH génétiquement modifiées produites à l'étape (i) dans un milieu contenant un inhibiteur de protéine kinase activée par le mitogène p38 (MAPK).

2. Procédé selon la revendication 1, dans lequel la manipulation génétique comprend la transduction d'un vecteur d'intégration ou dans lequel la manipulation génétique comprend l'édition génomique.

3. Procédé selon l'une quelconque des revendications 1 ou 2, dans lequel les CSH sont obtenues à partir d'un sujet, éventuellement dans lequel le sujet est un sujet humain.

4. Procédé selon la revendication 3, dans lequel les CSH sont des CSH adultes obtenues à partir de moelle osseuse ou de cellules sanguines périphériques du sujet humain, ou dans lequel les CSH sont obtenues à partir de cellules sanguines de cordon ombilical du sujet humain.

5. Procédé selon la revendication 2, dans lequel le vecteur d'intégration est un vecteur viral, éventuellement dans lequel le vecteur viral est un vecteur rétroviral ou un vecteur lentiviral.

6. Procédé selon la revendication 1 ou 2, dans lequel les CSH sont des CSH humains adultes.

7. Procédé selon l'une quelconque des revendications 6, dans lequel les CSH humains adultes sont obtenues à partir de la moelle osseuse ou de cellules sanguines périphériques.

8. Procédé selon l'une quelconque des revendications 1 à 7, dans lequel l'inhibiteur de MAPK est le doramapimod, le ralimétinib, un inhibiteur ATP compétitif à base d'aminopyridine de p38 MAPK ou un inhibiteur de pyridinylimidazole.

9. Procédé selon l'une quelconque des revendications 1 à 8, dans lequel la culture est effectuée pendant 1 à 7 jours.
